(19) Europäisches Patentamt / European Patent Office / Office européen des brevets

(11) **EP 3 015 111 B1**

(12) **EUROPEAN PATENT SPECIFICATION**

(45) Date of publication and mention
of the grant of the patent:
**10.01.2018 Bulletin 2018/02**

(21) Application number: **14817387.5**

(22) Date of filing: **26.06.2014**

(51) Int Cl.:
*A61K 36/898* (2006.01)    *A61K 36/889* (2006.01)
*A61K 36/71* (2006.01)    *A61K 36/70* (2006.01)
*A61K 36/48* (2006.01)    *A61K 36/315* (2006.01)
*A61K 36/19* (2006.01)    *A61K 33/30* (2006.01)
*A61K 33/26* (2006.01)    *A61K 33/06* (2006.01)
*A61P 1/00* (2006.01)    *A61P 37/06* (2006.01)
*A61K 36/47* (2006.01)    *A61K 36/33* (2006.01)
*A61K 36/185* (2006.01)    *A61K 36/65* (2006.01)
*A61K 36/899* (2006.01)

(86) International application number:
**PCT/CN2014/080812**

(87) International publication number:
**WO 2014/206310 (31.12.2014 Gazette 2014/53)**

(54) **USE OF CHINESE MEDICINE PREPARATION IN PREPARING DRUG FOR PREVENTING AND/OR TREATING CROHN'S DISEASE**

VERWENDUNG EINES CHINESISCHEN MEDIZINPRÄPARATS ZUR HERSTELLUNG EINES ARZNEISTOFFES ZUR VERHÜTUNG UND/ODER BEHANDLUNG VON MORBUS CROHN

UTILISATION D'UNE PRÉPARATION MÉDICINALE CHINOISE DANS LE CADRE DE LA PRÉPARATION D'UN MÉDICAMENT PERMETTANT DE PRÉVENIR ET/OU DE TRAITER LA MALADIE DE CROHN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2013 CN 201310259296**

(43) Date of publication of application:
**04.05.2016 Bulletin 2016/18**

(73) Proprietor: **Tasly Pharmaceutical Group Co., Ltd.**
**Tianjin 300410 (CN)**

(72) Inventors:
• **NIE, Zhaohong**
**Tianjin 300410 (CN)**

• **ZHAO, Min**
**Tianjin 300410 (CN)**

(74) Representative: **Gulde & Partner**
**Patent- und Rechtsanwaltskanzlei mbB**
**Wallstraße 58/59**
**10179 Berlin (DE)**

(56) References cited:
**EP-A1- 1 484 065    CN-A- 1 202 361**
**CN-A- 1 202 361    CN-A- 1 376 489**
**CN-A- 1 509 733    CN-A- 1 682 848**
**CN-A- 102 058 780    CN-A- 102 293 842**
**CN-A- 102 716 383**

**Description**

**FIELD OF INVENTION**

The present invention relates to the field of Traditional Chinese medicine (TCM). More specially, the invention relates to a use of traditional Chinese medicinal in preparation for the prevention or treatment of Crohn's disease.

**BACKGROUD OF THE INVENTION**

Crohn's disease is a kind of inflammatory bowel disease. Usually, a serie of signals occur in patients' colon, small intestine or stomach, e.g. inflammation, hyperemia or lymphatic swelling. The main difference from another inflammatory bowel disease, the ulcerative colitis (UC), lies in inflammationary position and inflammation itself. Histopathologically, any segment of digestive system may be affected, e.g. the small intestine, colon, stomach and esophagus, and the common sites include the terminal ileum and adjacent colon segment and right-half colon. UC, however, just occurs in colon and rectum, which is common in rectum and sigmoid. Microscopically, Crohn's disease may affect whole inner wall of bowel, while UC is restricted to mucosa.

Crohn's disease is a chronic and recurrent disease. Due to unknown cause, effectively theraputical drugs have not yet been developed. By now, the drugs used for treating Crohn's disease mainly include glucocorticoid, salicylic acid, immunosuppressive agents, antibiotics, methotrexate and biological agents (e.g. infliximab). Although these drugs are proven to have the effect to change the natural process of disease, they can not completely alleviate the conditions of disease and decrease the incidence of complications. Moreover, the chemicals of glucocorticoid and immunosuppressive agents often cause obvious adverse reaction and longtime administration will likely result in damage to the body.

In this case, the technical staff in prior arts hopes to develop effective traditional Chinese medicine preparations used for treating Crohn's disease by means of TCM technique. For example, Chinese patent (CN 102716383A) disclosed a TCM composition prepared with 15 raw medicines in accordance with formula. Because there were a lot of raw medicines in the composition, they had too complicated presceription-compounding to make it conducive to industrial production. Chinese patent (CN 102048728A) disclosed a use of artemisinin derivatives in preparation of medicine for treating Crohn's disease. However, as disclosed in the specification, the artemisinin derivatives were used for maintain treatment. That is to say, the artemisinin derivatives were not able to cure Crohn's disease radically.

Chinese patent (CN 1192787C) disclosed a TCM preparation used for treating UC. By further research, it is first discovered that the TCM preparation has an excellent effect on Crohn's disease with no significant adverse reaction.

**CONTENT OF THE INVENTION**

The objective of present invention is to provide a use of a TCM preparation in preparation of medicine for preventing and/or treating Crohn's disease. The preparation comprises the raw materials as 1-10 wt % of Sanguis Draxonis (Xuejie), 15-40 wt % of Radix Paeoniae Rubra (Chi Shao), 1-20 wt % of Indigo Naturalis (Qing Dai), 1-10 wt % of Halloysitum Rubrum (Chi Shi Zhi), 15-40 wt % of Catechu (Er Cha), 1-10 wt % of Calcined Alumen (Ku Fan), 5-30 wt % of Rhizoma Bletillae (Bai Ji) and 1-10 wt % of Calamina (Lu Gan Shi).

Another objective of present invention is to provide a use of the TCM preparation in preparation of medicine for preventing and/or treating extraintestinal manifestations and complications of Crohn's disease, thus ultimately improving extraintestinal manifestations and complications of Crohn's disease. The preparation comprises the raw materials as 1-10 wt % of Sanguis Draxonis (Xuejie), 15-40 wt % of Radix Paeoniae Rubra (Chi Shao), 1-20 wt % of Indigo Naturalis (Qing Dai), 1-10 wt % of Halloysitum Rubrum (Chi Shi Zhi), 15-40 wt % of Catechu (Er Cha), 1-10 wt % of Calcined Alumen (Ku Fan), 5-30 wt % of Rhizoma Bletillae (Bai Ji) and 1-10 wt % of Calamina (Lu Gan Shi).

Another objective of present invention is to provide a method for preventing and/or treating Crohn's disease. The method comprises administering to a patient in need thereof a preventive and/or therapeutic amount of the TCM preparation or a medicine composition comprising the TCM preparation.

According to present invention, the TCM preparation is competent to treating the Crohn's disease as long as its dose reaches effective dose, once or several times per day, preferably once per day. When the TCM preparation is used for preventing the Crohn's disease, its dose may be conditionally reduced.

**DESCRIPTION OF THE DRAWINGS**

Fig.1 showed the concentration-lethality curve of TCM preparation.

Fig.2 showed the zebra fish intestine in treatment group, the Crohn's disease model group and blank control group.

Fig.3 indicated the improving effect on bowel dilation in the Crohn's disease group administered with each tested drugs.

Fig. 4 showed the therapeutic efficency of each tested drugs on basis of the bowel area in the Crohn's disease group.

Fig.5 showed the distribution of neutrophil in intestinal tissue in tested drug groups, the Crohn's disease model group and control group. (The region shown by gray line was the zebra fish intestine and the shown by white bright spot was the neutrophil.)

Fig.6 showed the effect on the number of neutrophil in intestinal tissue in the Crohn's disease model group administered with each tested drugs.

Fig.7 showed the effect on regression of inflammation in the Crohn's disease group administered with each tested drugs.

Fig.8 showed the intestinal tissue slice in tested drug group, the Crohn's disease model group and control group. (The arrows indicated the zebra fish intestine, magnification of left and right images were respectively $20 \times$ and $40 \times$.)

Fig.9 showed the effect on the disease active index (DAI) scores in TNBS model rats administered intracolonically with the TCM preparation **j** (namely, the low-dose, middle-dose and high-dose group).

Fig. 10 showed the effect on the disease active index (DAI) scores in TNBS model rats administered intragastrically with the TCM preparation **j** (namely, the low-dose, middle-dose and high-dose group).

Fig.11 showed the effect on the serum content of IFN-$\gamma$, IL-1, IL-4, IL-10 and intestinal mocusa Gln in TNBS model rats administered intracolonically with the TCM preparation **j** (namely, the low-dose, middle-dose and high-dose group).

Fig. 12 showed the effect on the serum content of IFN-$\gamma$, IL-1, IL-4, IL-10 and Gln in TNBS model rats administered intragastrically with the TCM preparation **j** (namely, the low-dose, middle-dose and high-dose group).

Fig.13 indicated the pathological pictures in rats of the blank control group, model group, positive control group and tested drug groups administered intracolonically with the TCM preparation **j** (namely the low-dose, middle-dose and high-dose group).

Fig. 14 indicated the pathological pictures in rats of the blank control group, model group, positive control group and tested drug groups administered intragastrically with the TCM preparation **j** (namely the low-dose, middle-dose and high-dose group).

Fig. 15 showed the effect on the DAI scores in OXZ model rats administered intragastrically with the TCM preparation **k** (namely, the low-dose, middle-dose and high-dose group).

Fig.16 showed the effect on the DAI scores in OXZ model rats administered intracolonically with the TCM preparation **k** (namely, the low-dose, middle-dose and high-dose group).

Fig.17 showed effect on the serum content of IFN-$\gamma$, IL-4 and intestinal mucosa Gln in OXZ model rats administered intracolonically with the TCM preparation **k** (namely, the low-dose, middle-dose and high-dose group).

Fig.18 showed effect on the serum content of IFN-$\gamma$, IL-4 and intestinal mucosa Gln in OXZ model rats administered intragastrically with the TCM preparation **k** (namely, the low-dose, middle-dose and high-dose group).

Fig.19 indicated the pathological pictures in rats of the blank control group, model group, positive control group and tested drug groups administered intracolonically with the TCM preparation **k** (namely the low-dose, middle-dose and high-dose group).

Fig.20 indicated the pathological pictures in rats of the blank control group, model group, positive control group and tested drug groups administered intrarectally with the TCM preparation k (namely, the low-dose, middle-dose and high-dose group).

## DETAILED DESCRIPTION OF THE INVENTION

In one embodiment, the present invention relates to a use of a TCM preparation in preparation of medicine for preventing and/or treating the Crohn's disease. The preparation comprises the raw materials as 1-10 wt % of Sanguis Draxonis (Xuejie), 15-40 wt % of Radix Paeoniae Rubra (Chi Shao), 1-20 wt % of Indigo Naturalis (Qing Dai), 1-10 wt % of Halloysitum Rubrum (Chi Shi Zhi), 15-40 wt % of Catechu (Er Cha), 1-10 wt % of Calcined Alumen (Ku Fan), 5-30 wt % of Rhizoma Bletillae (Bai Ji) and 1-10 wt % of Calamina (Lu Gan Shi).

Preferably, the preparation comprises the raw materials as 3-7 wt % of Sanguis Draxonis, 25-35 wt % of Radix Paeoniae Rubra, 5-15 wt % of Indigo Naturalis, 1-5 wt % of Halloysitum Rubrum, 25-35 wt % of Catechu, 1-5 wt % of Calcined Alumen, 10-20 wt % of Rhizoma Bletillae and 1-5 wt % of Calamina.

Mostly preferably, the preparation comprises the raw materials as 5.1 wt % of Sanguis Draxonis, 30.3 wt % of Radix Paeoniae Rubra, 10.1 wt % of Indigo Naturalis, 3 wt % of Halloysitum Rubrum, 30.3 wt % of Catechu, 3 wt % of Calcined Alumen, 15.2 wt % of Rhizoma Bletillae and 3 wt % of Calamina.

In another embodiment, the present invention relates to a use of the TCM preparation in preparation of medicine for preventing and/or treating extraintestinal manifestations and complications of the Crohn's disease.

In preferable embodiment, the complications of the Crohn's disease include one or more kinds of intestinal obstruction, intestinal fistula, lower gastrointestinal massive hemorrhage, intestinal perforation, septicemia, abdominal abscess and anal diseases.

In another preferable embodiment, the extraintestinal manifestations of the Crohn's disease include oral mucositis, e.g. the aphthous ulcer; skin pathological changes, e.g. the erythema nodosum (EN), the pyoderma gangrenosum (PG) and non-specific rash; eye pathological changes, e.g. the iridocyclitis and conjunctivitis; arthral lesion, e.g. the arthral pain and arthritis; hepatobiliary lesion, e.g. abnormal liver function and gallstone.

According to present invention, the TCM preparation can be prepared into any one of pharmaceutically acceptable dosage forms, including : enema; tablet, e.g. the suger-coated tablet, film-coated tablet and enteric-coated tablets; capsule, e,g, the hard capsule and soft capsule; oral lquid; buccal tablet; granule; instant powder; pill; dripping pill; pulvis, paste, e.g. the ointment and paster; Dan; suspension; powder; solution, e.g. the injection; suppository; cream; spray; dry powder inhalation; aerosol; drop; lozenge and patch.

According to present invention, the enema is prepared by the method disclosed in Chinese Patent (CN1192787C). The dosage forms of tablet, capsule, oral liquid, buccal tablet, granule, instant powder, pill, dripping pill, pulvis, paste, Dan, suspension, powder, solution, suppository, cream, spray, dry powder inhalation, aerosol, drop, lozenge and patch may be prepared by the conventional methods known in prior arts.

According to present invention, the enema includes conventional carriers, e.g. the diluent, lubricant and wetting agent etc.

According to present invention, the orally-administrated dosage forms of TCM preparations include conventional carriers, e.g. one or more kinds of the adhersive, filling agent, diluent, tableting agent, lubricant, disintegrating agent, colorant agent, flavoring agent, wetting agent. If necessary, the tablet may be coated.

According to present invention, the filling agents include cellulose, mannitol, lactose and other analogous filling agent. Competent disintegrating agents include starch, polyvinylpyrrolidone (PVP) and starch derivative (e.g. sodium hydroxyethyl starch). Competent lubricants include magnesium stearate. Competently pharmaceutically acceptable wetting agents include sodium dodecyl sulfate.

According to present invention, oral solid preparations of TCM preparations can be prepared by a conventional method of blending, filling, tabletting or granulating etc. Blending is repeated to make the active pharmaceutical ingredients (API) distributed uniformly in the final dosage form.

According to present invention, oral liquid preparations are in dosage form of either water-soluble or oil-soluble suspension, solution, emulsion, syrup or elixir, or drying powder that is always reconstituted with water or other suitable solvent before clinical use. The oral liquid preparation may contain conventional excipients, e.g. suspending agent, such as the sorbitol, syrup, methylcellulose, gelatin, hydroxy ethyl cellulose, hydroxy methyl cellulose, aluminum stearate gel or hydrogenated edible fat; emulsifying-agent, e,g lecithin, sorbitan monoleate or arabic gum; non-aqueous excipient (including edible oil) e.g. almond oil, fractionated coconut oil, oil ester, propylene glycol or ethanol as well as preservative e.g. methylparaben, nipasol, sorbic acid. If necessary, conventional flavoring agent or colorant agent can be included.

According to present invention, as for injections, the preparation unit contains the TCM preparation as API and aseptic excipients. Whether the API is dissolved or suspended in the liquid depends on the type and concentration of excipients. Generally, solution is prepared by dissolving the TCM preparation in the excipients as the API, sterilizing, loading into an appropriate vial or ampoule and sealing. Some pharmaceutically acceptable adjuvant, e.g. local anaesthetic, preservative and buffering agent can be added as required. In order to improving its stability, before loaded into the vial, this TCM preparation of present invention can be frozen and treated in vacuum to remove water.

According to present invention, the TCM preparation can be prepared by optionally adding pharmaceutically acceptable excipients. The excipients are selected, as follows: sugar alcohol, e.g. mannitol, sorbitol, xylitol; amino acid, e.g. cysteine hydrochloride, methionine, glycine, Vitamin C; EDTA disodium, EDTA calcium disodium salt; inorganic salt, e.g. one valence alkali carbonate, acetate, phosphate or its aqueous solution, sodium chloride, potassium chloride, sodium pyrosulfite, sodium bisulfite, sodium thiosulfate; calcium carbonate, calcium bicarbonate; stearate, e.g. calcium stearate, magnesium stearate; inorganic acid, e.g. hydrochloride, sulfate, phosphoric acid; organic acid, e.g. acetic acid; organic acid salt, e.g. sodium lactate; polysaccharide, cellulose and its derivatives, e.g. maltose, glucose, fructose, dextran, sucrose, lactose, cyclodextrin (β-cyclodextrin), starch, mercaptoacetic acid; silicon derivative; alginate; gelatin; PVP, glycerol; Tween-80, agar gel; surfactant; phospholipids; Kaolin; talcum powder etc.

According to present invention, use and dose of the TCM preparation depend on the condition of patients in clinic. In preferable example, the TCM preparation is used in dosage form of enema for treating the Crohn's disease, administered to pateints once or several times per day, preferably once per day; 60~80ml per time, preferably 80ml per time. Dose of the TCM preparation may be reduced conditionally, when using it for preventing the Crohn's disease, for example, 1/2, 1/3 and 1/4 of therapeutically effective dose.

## EMBODIMENTS

Techinical scheme of present invention will he further elaborated by following preparative examples and trial examples.

Herein, the TCM preparations are prepared by the method disclosed in Chinese patent (CN 1192787 C).

Preparative example 1

The raw materials by following weight were used to prepare the TCM preparation **a.**

14g of Sanguis Draxonis, 84g of Radix Paeoniae Rubra, 28g of Indigo Naturalis, 8.4g of Halloysitum Rubrum, 84g of Catechu, 8.4g of Calcined Alumen, 42g of Rhizoma Bletillae and 8.4g of Calamina.

Preparative example 2

The raw materials by following weight were used to prepare the TCM preparation **b.**

22.1g of Sanguis Draxonis, 69g of Radix Paeoniae Rubra, 25g of Indigo Naturalis, 25g of Halloysitum Rubrum, 69g of Catechu, 17g of Calcined Alumen, 28g of Rhizoma Bletillae and 22.1g of Calamina.

Preparative example 3

The raw materials by following weight were used to prepare the TCM preparation **c.**

19g of Sanguis Draxonis, 67g of Radix Paeoniae Rubra, 27.1g of Indigo Naturalis, 19g of Halloysitum Rubrum, 67g of Catechu, 14.1g of Calcined Alumen, 47g of Rhizoma Bletillae and 17g of Calamina.

Preparative example 4

The raw materials by following weight were used to prepare the TCM preparation **d.**

11g of Sanguis Draxonis, 91g of Radix Paeoniae Rubra, 19g of Indigo Naturalis, 14g of Halloysitum Rubrum, 91g of Catechu, 12.2g of Calcined Alumen, 28g of Rhizoma Bletillae and 11g of Calamina

Preparative example 5

The raw materials by following weight were used to prepare the TCM preparation **e.**

8g of Sanguis Draxonis, 97g of Radix Paeoniae Rubra, 14g of Indigo Naturalis, 6g of Halloysitum Rubrum, 97g of Catechu, 2.2g of Calcined Alumen, 47g of Rhizoma Bletillae and 6g of Calamina.

Preparative example 6

The raw materials by following weight were used to prepare the TCM preparation **f.**

3g of Sanguis Draxonis, 42g of Radix Paeoniae Rubra, 16g of Indigo Naturalis, 4g of Halloysitum Rubrum, 45g of Catechu, 4g of Calcined Alumen, 13g of Rhizoma Bletillae and 5g of Calamina Preparative example 7

The raw materials by following weight were used to prepare the TCM preparation **g.**

13g of Sanguis Draxonis, 56g of Radix Paeoniae Rubra, 23g of Indigo Naturalis, 9g of Halloysitum Rubrum, 58g of Catechu, 12g of Calcined Alumen, 29g of Rhizoma Bletillae and 9g of Calamina.

Preparative example 8

The raw materials by following weight were used to prepare the TCM preparation **h.**
20g of Sanguis Draxonis, 71g of Radix Paeoniae Rubra, 35g of Indigo Naturalis, 13g of Halloysitum Rubrum, 66g of Catechu, 19g of Calcined Alumen, 47g of Rhizoma Bletillae and 13g of Calamina.

Preparative example 9

The raw materials by following weight were used to prepare the TCM preparation **i.**
23g of Sanguis Draxonis, 95g of Radix Paeoniae Rubra, 44g of Indigo Naturalis, 20g of Halloysitum Rubrum, 83g of Catechu, 23g of Calcined Alumen, 68g of Rhizoma Bletillae and 19g of Calamina.

Preparative example 10

The raw materials by following weight were used to prepare the TCM preparation **j.**
27g of Sanguis Draxonis, 110g of Radix Paeoniae Rubra, 56g of Indigo Naturalis, 26g of Halloysitum Rubrum, 105g of Catechu, 27g of Calcined Alumen, 81g of Rhizoma Bletillae and 25g of Calamina.

Preparative example 11

The raw materials by following weight were used to prepare the TCM preparation **k.**
1g of Sanguis Draxonis, 16g of Radix Paeoniae Rubra, 1g of Indigo Naturalis, 1g of Halloysitum Rubrum, 16g of Catechu, 1g of Calcined Alumen, 3g of Rhizoma Bletillae and 1g of Calamina.

Preparative example 12

The raw materials by following weight were used to prepare the TCM preparation **l.**
17g of Sanguis Draxonis, 40g of Radix Paeoniae Rubra, 20g of Indigo Naturalis, 10g of Halloysitum Rubrum, 40g of Catechu, 10g of Calcined Alumen, 30g of Rhizoma Bletillae and 10g of Calamina.

Preparative example 13

The raw materials by following weight were used to prepare the TCM preparation **m.**
6g of Sanguis Draxonis, 94g of Radix Paeoniae Rubra, 20g of Indigo Naturalis, 10g of Halloysitum Rubrum, 40g of Catechu, 10g of Calcined Alumen, 70g of Rhizoma Bletillae and 10g of Calamina.

**Trial example 1** preliminary study of concentration for efficacy evaluation in zebra fish of the Crohn's disease model

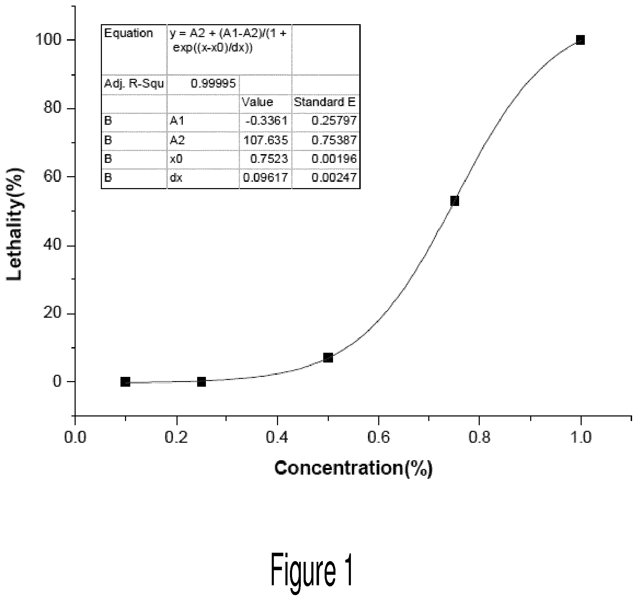

Fish farm water of wild-type AB strain zebra fish Crohn's disease model (1L reverse osmosis water added with 200mg instant sea salt with the conductivity of 480~510$\mu$S/cm, pH value of 6.9~7.2 and hardness of 53.7~71.6mg/L $CaCO_3$) was added with the TCM preparation (**a**) prepared by the method of EXAMPLE 1 in the concentration of 0.1%, 0.25%, 0.5%, 0.75% and 1% by volume. At the same time, the control group was given. At each concentration of the preparation (**a**), 30 zebra fish were treated, during which zebra fish death number was counted daily and the dead fish was removed in time. The relationship between the concentration of TCM preparation and lethality was seen in table 1, and the concentration-lethality curve present in Fig.1.

Table 1: Statistical relationship between concentration of preparation a and lethality

| Concentration of preparation **a** (%) | Dearth number | Lethality (%) |
|---|---|---|
| 0.10 | 0 | 0 |
| 0.25 | 0 | 0 |
| 0.50 | 2 | 7 |
| 0.75 | 16 | 53 |
| 1.00 | 30 | 100 |

By using OriginPro 8.0 software, MNLC (Maximam Non-lethal concentration) of the TCM preparations was calculated as 0.3% by volume.

**Trial example 2** efficacy in zebra fish of the Crohn's disease model

According to the results of concentration explored preliminarily in Trial example 1, there were 3 levels set in treatment group to evaluate the efficacy in zebra fish Crohn's disease model, respectively in volume ratio of 1/10 MNLC (0.03%), 1/3 MNLC (0.1%) and MNLC (0.3%) (Hereafter abbreviated as "treatment group 1 of preparation **a**", "treatment group 2 of preparation **a**" and "treatment group 3 of preparation **a**"). Meanwhile, the positive control group, blank control group, Crohn's disease model group and excipient control group were set in the research. In the positive control group, the prednisolone was finally at concentration of $10\mu M$ in fish farm water. The enema excepient disclosed in Chinese patent (CN 1192787 C) was used in the excepient control group, including adding PEG-400 solutions containing tragacanth and potassium sorbate into the fish farm water in the volume ratio of 0.3%. Wherein, the PEG-400 solution was prepared following method: adding 1.5g of tragacanth and 2g of potassium sorbate (particle size of $150\mu m$) into 28ml PEG.

Zebra fish Crohn's disease model was established with TNBS (2,4,6-trinitrobenzenesulfonic acid), mainly exhibiting inflammation. 30 zebra fish were randomly given to each group. 48 hours after respectively being treated with aforethe TCM preparation **(a)** in treatment groups (above-mentioned three concentrations), aforethe prednisolone in the positive control group and aforethe excipient in the excipient control group, 10 fish in each group were randomly selected to observe and photograph. The image analysis software was used to analyze the pictures. Meanwhile, intestine mocuscal thickness, intestinal diameter and intestinal area were observed carefully under the microscope and the intestinal diameter and intestinal area analyzed quantitatively. Theraputical ratio of tested drug in zebra fish Crohn's disease was calculated in accordance with the intestinal area in each group. The calculation formula was present as follows:

$$\text{Theraputical ratio (\%)}=[1\text{-(treatment group-blank control group)/(model group-blank control group)}]\times 100\%$$

Statistically analyzed results were expressed as mean $\pm$ SE. Variance analysis was used for comparison among many groups and Dunnett's T test for comparison between two groups. P<0.05 showed a statistically significant difference.

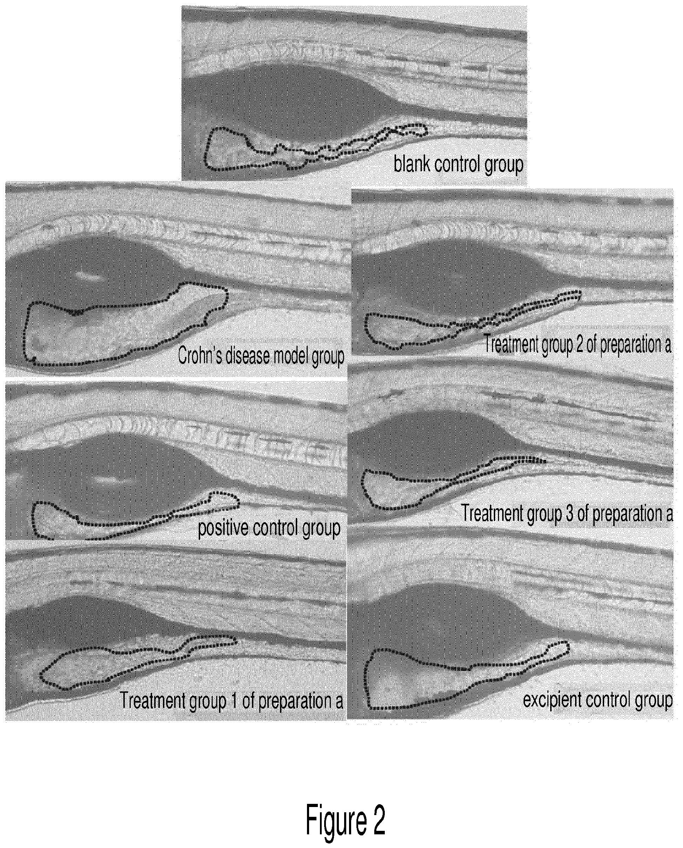

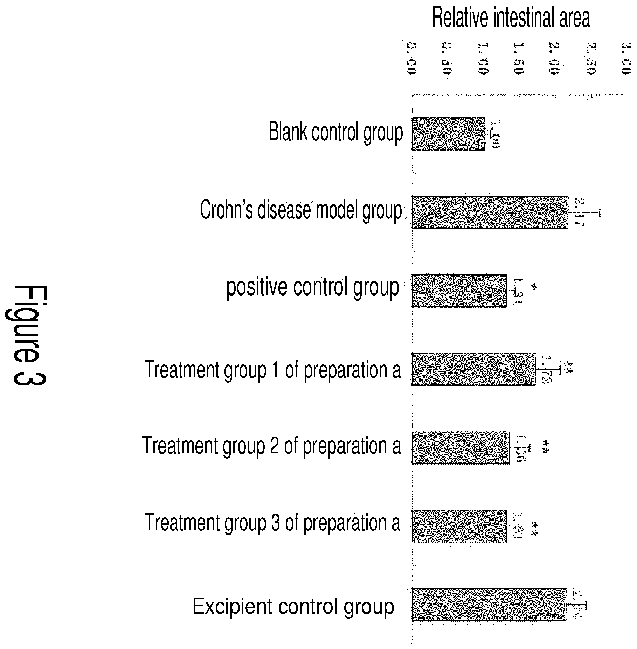

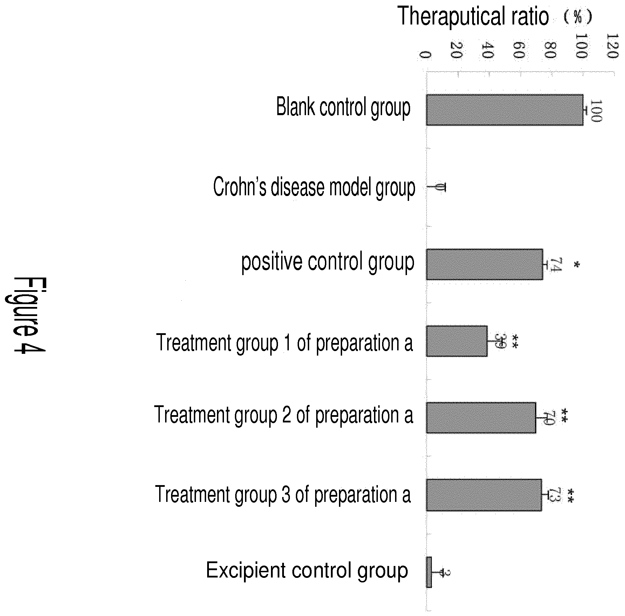

As shown in Fig.2~Fig.4, the intestinal mocusa was integrated and the folds clear in the blank control group. In the model group (Crohn's disease), zebra fish bowel was dilated (expressed by ratio of bowel area between the model group and the blank control group, the same below), the bowel area increased, the intestinal mocusa thinned and the folds vanished. In the positive control group, zebra fish bowel dilation was reduced, the bowel area decreased and the intestinal folds recovered significantly. In treatment group 2 and treatment group 3 of TCM preparation **(a)** (respectively administered with preparation **(a)** at 0.1% and 0.3%), zebra fish was dilution was improved significantly, the intestinal area approached the normal value and intestinal folds recovered basically. In the excipient control group, no theraputical effect was found in zebra fish Crohn's disease.

The prednisolone in the positive control group had theraputical ratio of 74 $\pm$ 3.40% in zebra fish Crohn's disease model, having statistically significant difference (p<0.01) in comparison with the model group. Partial theraputical ratio of 39 $\pm$ 9.30% was found in the treatment group 1 of preparation **(a)** at 0.3%. The treatment group 2 and 3 of preparation **(a)** had theraputical ratio of 70$\pm$7.49% and 73$\pm$4.81% respectively in zebra fish Crohn's disease model, having extremely significant difference (p<0.001) in comparison with the model group.

Theraputical ratio of tested drugs in zebra fish Crohn's disease model was seen in table2.

Table 2 theraputical ratio of tested drugs in zebra fish Crohn's disease model

| Groups | Intestinal area | | Intestinal diameter | | Therapeutical ratio (%) (Mean±SE) |
|---|---|---|---|---|---|
| | Intestinal area (Mean±SD) | Ratio of blank control group (Mean±SE) | Intestinal diameter (Mean±SD) | Ratio of blank control group (Mean±SE) | |
| Blank control group | 37230±5249 | 1.00±0.03 | 134±11 | 1.00±0.03 | - |
| Model group | 80867±16370 | 2.17±0.14 | 206±15 | 1.53±0.04 | - |
| Positive control group | 48646±4685* | 1.31±0.04* | 137±11* | 1.02±0.03* | 74±3.40* |

(continued)

| Groups | Intestinal area | | Intestinal diameter | | Therapeutical ratio (%) (Mean±SE) |
|---|---|---|---|---|---|
| | Intestinal area (Mean±SD) | Ratio of blank control group (Mean±SE) | Intestinal diameter (Mean±SD) | Ratio of blank control group (Mean±SE) | |
| Excipient control group | 74635±10653 | 2.14±0.09 | 194±10 | 1.45±0.02 | 3±7.73 |
| Treatment group 1 of preparation **a** | 64036±12872** | 1.72±0.11** | 185±21** | 1.38±0.05** | 39±9.33** |
| Treatment group 2 of preparation **a** | 50532±10328** | 1.36±0.09** | 143±16** | 1.06±0.04** | 70±7.49** |
| Treatment group 3 of preparation **a** | 48955±6628** | 131±0.06** | 131±27** | 0.97±0.06** | 73±4.81** |
| Noted: compared with the model group, *p<0.01, **p<0.001. | | | | | |

As shown in above table, the TCM preparation had excellent therapeutical effect on Crohn's disease, and even at low concentration it showed theraputical effect on Crohn's disease.

**Trial example 3** effect on regression of inflammation in zebra fish Crohn's disease model

Method was the same as the one in **Trial example** 2, except that the transgenic neutrophil fluorescent zebra fish was adopted. The TCM preparation (a) prepared by the method of Example 1 was added into the fish farm water in the transgenic neutrophil fluorescent zebra fish Crohn's disease model in the volume ratio of 0.03%, 0.1% and 0.3% to evaluate the effect of the preparation on inflammation regresssion in zebra fish Crohn's disease model.

The image analysis software was used to quantitatively analyse distribution of neutrophil in inflammationary tissue. Inflammation regression ratio of tested drugs in zebra fish Crohn's disease model was calculated on the basis of the number of neutrophil in bowel. The formula calculated for inflammation regression was present as follows:

Inflammation regression ratio (%)=[1-(treatment group-blank control group)/(model group-blank control group)]×100%

Statistically analyzed results were expressed as mean±SE. Variance analysis and Dunnett's T test were used. P<0.05 showed a statistically significant difference.

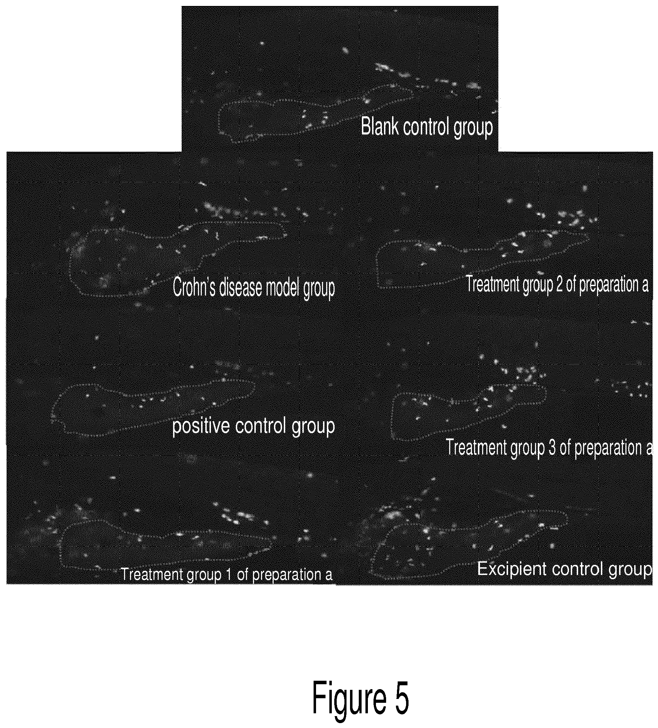

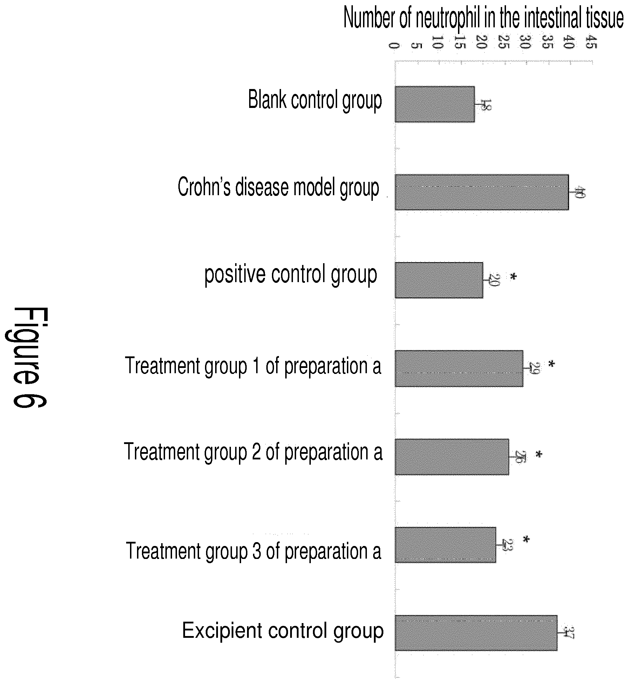

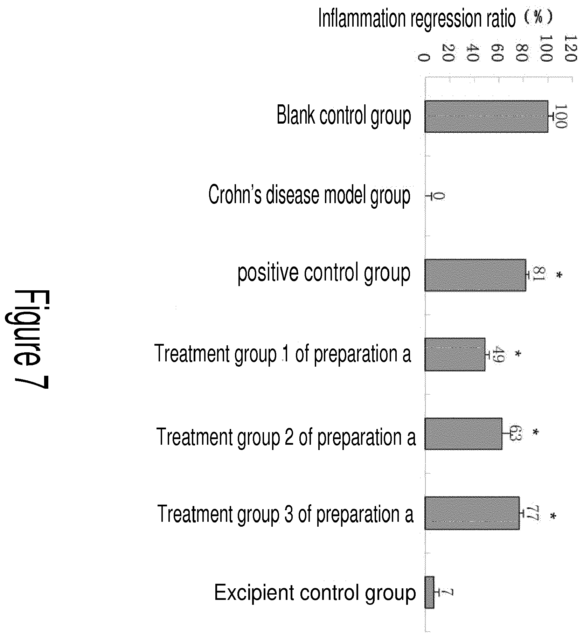

As shown in Fig.5~Fig.7, a few neutrophils were found in the intestinal tissue of zebra fish in the blank control. The bowel was dilated obviously, and a large number of neutrophils infiltration observed in the intestinal tissue in the Crohn's disease model, showing significant inflammation. In the positive control group (prednisolone), the number of neutrophil was reduced significantly in the intestinal tissue. In treatment groups 1, 2 and 3 of TCM preparation **(a),** the number of neutrophil was reduced. There were still a large number of neutrophils gathering in the the intestinal tissue of the excipient control group.

The prednisolone in the positive control group had the inflammationary regression ratio of 81 ± 2.48%, having statistically significant difference (p<0.01) compared with the model group. The treatment groups 1, 2 and 3 of preparation **(a)** had theraputical ratio of 49±3.50%, 63±6.48% and 77±3.50% respectively, each group having statistically extremely significant difference (p<0.001) compared with the model group.

Inflammation regression ratio of tested drugs in zebra fish Crohn's disease model was seen in table3.

Table 3 Inflammation regression ratio of tested drugs in zebra fish Crohn's disease model

| Groups | Number of neutrophil (Mean±SD) | Ratio of the blank control group (Mean±SE) | Inflammation regression ratio (%) (Mean±SE) |
|---|---|---|---|
| Blank control group | 18±2 | 1.00±0.05 | - |

(continued)

| Groups | Number of neutrophil (Mean±SD) | Ratio of the blank control group (Mean±SE) | Inflammation regression ratio (%) (Mean±SE) |
|---|---|---|---|
| Model group | 40±3 | 2.20±0.06 | - |
| Positive control group | 20±1* | 1.22±0.03* | 81±2.48* |
| Excipient control group | 37±2 | 2.11±0.05 | 7±3.79 |
| Treatment group 1 of preparation **a** | 29±2* | 1.61±0.04* | 49±3.50* |
| Treatment group 2 of preparation **a** | 26±4* | 1.44±0.08* | 63±6.48* |
| Treatment group 3 of preparation **a** | 23±2* | 1.28±0.04* | 77±3.50* |
| Noted: compared with the model group, **p<0.001. | | | |

As shown in above table, the TCM preparation had excellent effect of regressing inflammation on the Crohn's disease model, and even at low concentration it showed effect of regressing inflammation on Crohn's disease.

**Trial example 4** improving effect of histopathology in zebra fish Crohn's disease model

Method was the same as the one in Trial example 2. Drugs were administered to the Crohn's disease model. After being treated, the zebra fish were fixed with 4% paraformaldehyde and transferred to 70% ethanol. The intestinal tissue was determined by histopathological research after dehydration, embedding, sectioning and H&E dyeing.

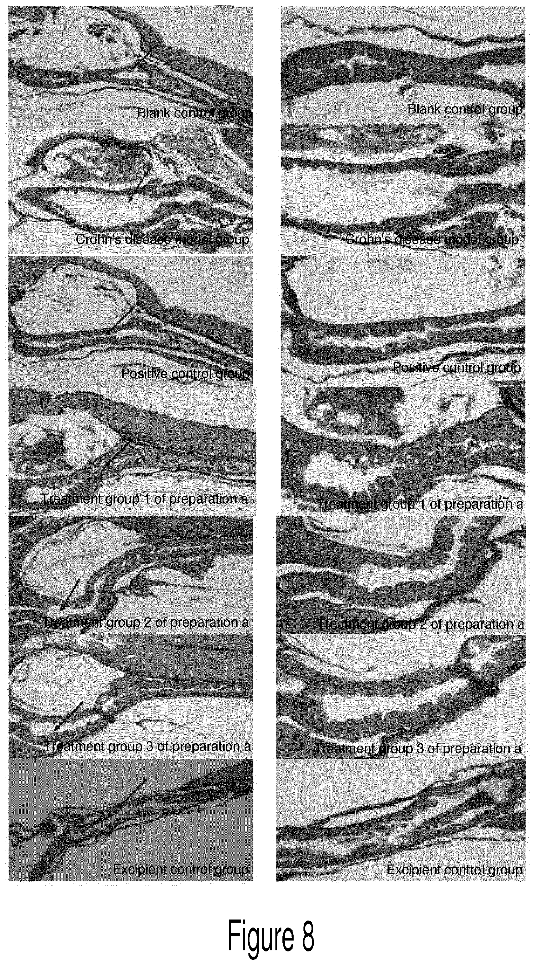

As shown in Fig.8, in the blank control group, zebra fish intestinal mucosa was smooth, integrated, the intestinal folds obvious and mucus secretion found in intestinal cavity. In the Crohn's disease model group, the bowel was dilated, the intestinal musoca thinned, the intestinal folds vanished and the mucosa erosive and ulcerated. In the positive control group (prednisolone), the intestinal mucosal histopathology was improved obviously. In treatment groups 1, 2 and 3 of TCM preparation **(a),** the intestinal area and folds were basically recovered, the intestinal mucosal inflammation improved significantly and no marked ulcer occured. No histopathological improvement was found in the excipient control group.

According to the results of above Trial Examples 2~4, the TCM preparation could effectively improve the intestinal thickness, reduce the intestinal dilation caused by inflammation, facilitate inflammationary regression and ameliorate in the Crohn's disease model. Compared with the Crohn's disease model, all quantitative evaluation indice (e.g. theraputical ratio and inflammation regression ratio) in the TCM preparations had statistically extremely significant difference (p<0.001). No therapeutic effects were found on the Crohn's disease in the excipeint control disease.

**Trial example 5** therapeutical effect of the TCM preparation j on TNBS-caused Crohn's disease model

1. Aminals: SD rats, half male and female, weighing 180~220g, were purchased from Aminal center, Institute of Hygiene and Environmental Medicine, Academy of Military Medical Sciences of PLA with the certificate Number SCXK-2009-003.

2. Drug and reagent: mesalamine, used as positive drug, was provided by Tasly Pharmaceutical Group Co. Ltd. 5% TNBS was purchased from Sigma Inc. IFN-γ, IL-1, IL-4, IL-10 and Gln Elisa assay kits were purchased from bioswamp Inc. Stool occult blood assay kit was provided by Nanjing Jiancheng Bioengineering Institute.

3. Apparatus: AB135-S electronic analytical scale was purchased from Mettle Toledo Inc. Switzerland.

Method:

Grouping: experiment was carried out by using two ways of intracolonical and intragastric administrations. Hence, rats were divided into following groups: the blank control intragastrical group, the model intragastrical group, the positive control intragastrical group, the TCM preparation j high-dose intragastrical group, the middle-dose intragastrical group, the low-dose intragastrical group, the blank control intracolonical group, the model intracolonical group, the positive control intracolonical group, the TCM preparation j high-dose intracolonical group, the middle-dose intracolonical group and the low-dose intracolonical group. There were 10 rats each group.

Method for establishing rat model: referring to Morris method, TNBS was used to induce the SD rat Crohn's disease model (abbreviated as "TNBS model"). 24 hours before modeling, the SD rats were fasted but water was given. After weighing, the SD rats were anesthetized by intraperitoneal injection of pentobarbital sodium. ① Prepration of TNBS enema liquid: 5% TNBS was mixed with anhydrous ethanol in the volume ratio of 2:1, loaded in bottle and stored at 4°C. ② Clystering: in the modeling group, rats were treated by clyster of TNBS solution in the ratio of 150mg/kg rat weight (namely 4.5ml/kg rat weight), and in the blank control group, the rats treated by clyster of 0.9% (w/v) normal saline in the same ratio. The enemator (a latex tube with outer diameter of 2mm) was inserted the anus 6~8cm inside, and the enema liquids injected. The rats were inverted by lifting tails for lmin to make TNBS fully take effect in intestinal cavity, preventing the solution from overflowing.

Administration method: after modeling, the TCM preparation **j** saline solutions at corresponding dose were administered to the groups of preparation **j** of all doses and the mesalamin saline solution, a positive drug, administrated to the positive control group. The rats of blank control group were normally fed with free access to water and the same volume of normal saline was given in the same administration routes by referring to the dosage regime of aforethe treatment groups. The rats of model group were administrated with the same volume of normal saline in the same administration routes. The dose of each group was as follows: mesalamin group at 100mg/kg rat weight, the TCM preparation **J** high-dose group at 2.023g raw medicine/kg rat weight, the middle-dose group at 1.012g raw medicine/kg rat weight and the low-dose group at 0.506g raw medicine/kg rat weight. In both intragastric and intracolonical routes, test drugs saline solution or normal saline were administrated in a ratio of 0.7ml/100g rat weight, once per day consecutively for 21 days.

After being treated as disclosed above, the rats in each group were evaluated by following indice.

(1) General Indice: weight change and stool (e.g. fecal occult blood, bloody stool and diarrhea) was observed everyday to calculate DAI (disease activatity index) score.

$$DAI\ score=(weight\ loss\ percentage+stool\ character\ fraction+\ hematochezia\ fraction)/3$$

Table 4 DAI score criteria

| Weight loss percentage (wt%) | Stool character | Fecal occult blood/gross blood stool | Score |
|---|---|---|---|
| 0% | Normal | Normal | 0 |
| 1%~5% | Slightly loose | Positive stool occult blood | 1 |
| 5%~10% | Loose | Increased positive stool occult blood | 2 |
| 10%~15% | Slightly watery stool | Gross blood stool | 3 |
| >15% | Watery stool | Increased gross blood stool | 4 |

(2) Serum indice: after final administration, the testing rats were fasted but given water. 24 hours later, blood was gained from abdominal arota. Serum IFN-$\gamma$, IL-1, IL-4, IL-10 and Gln in intestinal musoca were determined by enzyme-linked immunosorbent assay (ELISA) in rats.

(3) Phathological indice: the colon of rats was taken out. The gross colon tissue damage was scored, subsequently examined and scored histopathologically by conventional HE staining. The scoring criteria were seen in table 5.

Table 5 colon tissue damage and histopathological scoring criteria

| Score | Gross damage scoring criteria | Score | Histopathological scoring criteria |
|---|---|---|---|
| 0 | No damage | 0 | Normal colon tissue |
| 1 | Congestion and edema occurs but no ulcer | 1 | Inflammation or ulcer site occurs |
| 2 | Ulcer occurs but no significant inflammation | 2 | Inflammation or ulcer sites are restricted to mocusa and submocusa |
| 3 | Ulcer occurs but just one inflammation | 3 | Inflammation or ulcer sites invade muscularis propria |

(continued)

| Score | Gross damage scoring criteria | Score | Histopathological scoring criteria |
|---|---|---|---|
| 4 | More than 2 ulcers and inflammations occurs and ulcer stie<1cm | 4 | Transmural inflammation or ulcer sites invade serous layer |
| 5 | More than 2 ulcers and inflammations occurs, at leat one ulcer or inflammation site > 1cm | 5 | Transmural inflammation or large area of ulcer invades serous layer and perforates |

Statistical analysis: Spss 17.0 statistical software was used to analyze above scores. Data were expressed as Mean$\pm$SD and t-test used for comparison between groups. P$\leq$0.05 showed a statistically significant difference.
By intragastric administration or intracolonical administration, the results were seen following table 6~11.

(1) Results of general indice (DAI scores)

Table 6 effect of TCM preparation j by intracolonical administration on DAI scores in TNBS model (Mean±SD)

| Administration days | Blank control group | Model group | Positive control group (100mg/kg) | High-dose (2.023g/kg) | Middle-dose (1.012g/kg) | Low-dose (0.506g/kg) |
|---|---|---|---|---|---|---|
| 0th day | 0.000±0.000 | 0.000±0.000 | 0.000±0.000 | 0.000±0.000 | 0.000±0.000 | 0.000±0.000 |
| 1st day | 0.167±0.176## | 1.900±0.274** | 1.900±0.274** | 1.967±0.554** | 2.000±0.385** | 1.933±0.466** |
| 2nd day | 0.100±0.161## | 2.400±0.378** | 1.867±0.571**# | 1.100±0.353**## | 1.800±0.652**## | 1.633±0.637**## |
| 3rd day | 0.000±0.000## | 2.567±0.418** | 2.100±0.754** | 1.267±0.625**## | 2.037±0.633**# | 1.833±0.614**## |
| 4th day | 0.000±0.000## | 2.600±0.540** | 2.067±0.7217** | 1.200±0.670**## | 1.704±0.564**## | 2.067±0.783** |
| 5th day | 0.000±0.000## | 2.778±0.500** | 1.733±0.562**## | 1.000±0.444**## | 1.481±0.475**## | 2.167±0.774**# |
| 6th day | 0.000±0.000## | 2.667±0.500* | 1.367±0.532**## | 0.933±0.410**## | 1.481±0.530**## | 2.033±0.867**# |
| 7th day | 0.000±0.000## | 2.407±0.5725** | 1.267±0.467**## | 0.833±0.283**## | 1.333±0.408**## | 1.833±0.892**## |
| 8th day | 0.000±0.000## | 2.185±0.530** | 1.167±0.360**## | 0.733±0.306**## | 1.185±0.412**## | 1.556±0.726**## |
| 9th day | 0.000±0.000## | 2.042±0.486** | 1.067±0.306**## | 0.667±0.272**## | 1.037±0.261**## | 1.296±0.539**## |
| 10th day | 0.000±0.000## | 1.917±0.427** | 1.000±0.314**## | 0.633±0.331**## | 0.963±0.200**## | 1.259±0.494**## |
| 11th day | 0.000±0.000## | 1.750±0.427** | 0.933±0.211**## | 0.600±0.344**## | 0.963±0.200**## | 1.222±0.500**## |
| 12th day | 0.000±0.000## | 1.667±0.356** | 0.833±0.236**## | 0.533±0.358**## | 0.926±0.147**## | 1.111±0.471**## |
| 13th day | 0.000±0.000## | 1.500±0.178** | 0.833±0.236**## | 0.400±0.306**## | 0.926±0.147**## | 1.037±0.455**## |
| 14th day | 0.000±0.000## | 1.500±0.178** | 0.767±0.353**## | 0.300±0.292*## | 0.741±0.278**## | 0.926±0.401**## |
| 15th day | 0.000±0.000## | 1.500±0.178** | 0.733±0.344**## | 0.300±0.292*## | 0.630±0.200**## | 0.741±0.434**## |
| 16th day | 0.000±0.000## | 1.458±0.173** | 0.667±0.314**## | 0.267±0.263*## | 0.593±0.147**## | 0.741±0.434**## |
| 17th day | 0.000±0.000## | 1.417±0.236** | 0.600±0.263**## | 0.233±0.225*## | 0.593±0.147**## | 0.625±0.375**## |
| 18th day | 0.000±0.000## | 1381±0.230** | 0.567±0.274**## | 0.200±0.172## | 0.555±0.167**## | 0.542±0.396**## |
| 19th day | 0.000±0.000## | 1.333±0192** | 0.467±0.281**## | 0.133±0.72## | 0.481±0.294**## | 0.458±0.396**## |
| 20th day | 0.000±0.000## | 1.333±0.192** | 0.433±0.274**## | 0.033±0.105## | 0.370±0.309*## | 0.417±0.345**## |
| 21st day | 0.000±0.000## | 1.333±0.192** | 0.433±0.274**## | 0.033±0.105## | 0.333±0.289**## | 0.417±0.345**## |

Noted: compare with the blank control group, * P<0.05, ** P<0.01; compared with the model group, # P<0.05, ## P<0.01. Wherein, "on 0th day" refered to the DAI scores of rats 1 day before the TNBS model had been established.

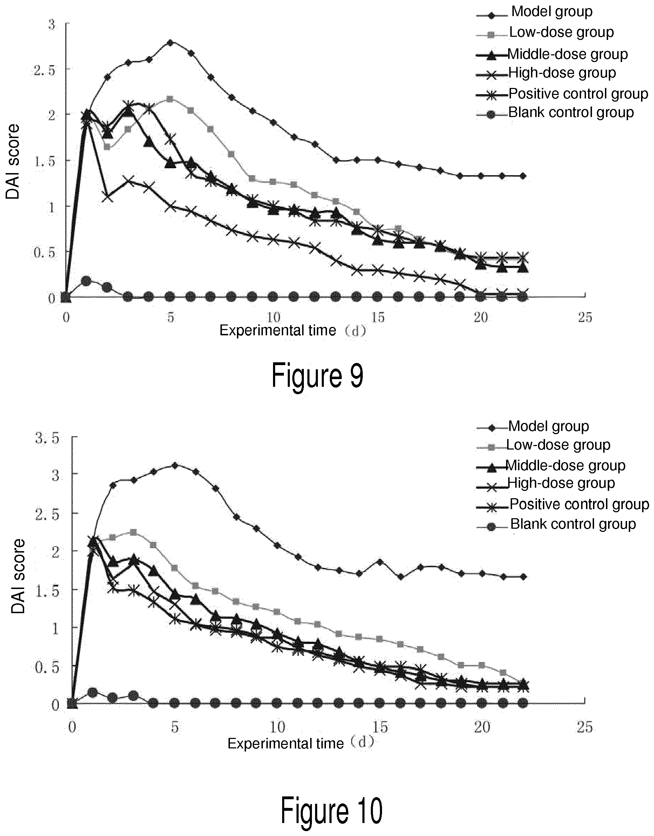

As shown in table 6 and Fig.9, in the blank control group, the symptoms of significant body weight loss, stool character abnormal change, e.g. diarrhea, bloody stool and positive stool occult blood had not been found in rats. In the model group, after intracolonical administration of TNBS, the gross blood stool was observed on $1^{st}$ day (namely taking normal saline), which was accompanied with body weight loss. These syptomes were lasted for 6~8 days. On $9^{th}$ day, after administrating normal saline, the stool of rats in this group gradually showed positive stool occult blood, watery stool and gradual body weight increase. DAI scores in the model group were much higher than the one in the blank control group, showing a statistically significant difference. In treatment groups of TCM preparation **j** of all doses and positive control group, although being treated with tested drugs after intracolonical administration of TNBS, the rats showed body weight loss within previous 6~8 days after administration. Compared with the model group, the body weight in aforethe groups of all doses and positive control group was decreased to a lesser degree. Actually, degree of body weight loss was gradually improved with increase of administration time. Intially, the stool character mainly included positive stool occult blood with no gross blood stool. Until $21^{st}$ day after administration, the stool character in most of rats was recovered except slightly positive stool occult blood in few rats. Wherein, the TCM preparation **J** high-dose group had the most obvious improving effect. There were much lower DAI scores in the TCM preparation **J** group of all doses and the positive control group than the one in model group, having stastically significant difference ($p<0.01$).

Table 7 effect of TCM preparation j by intragastric administration on DAI scores in TNBS model rats (Mean±SD)

| Administration days | Blank control group | Modeling group | Positive control group (100mg/kg) | High-dose (2.023g/kg) | Middle-dose (1.012g/kg) | Low-dose (0.506g/kg) |
|---|---|---|---|---|---|---|
| 0th day | 0.000±0.000 | 0.000±0.000 | 0.000±0.000 | 0.000±0.000 | 0.000±0.000 | 0.000±0.000 |
| 1st day | 0.133±0.172## | 2.133±0.172** | 2.000±0.373** | 2.133±0.281** | 2.133±0.391** | 2.000±0.314** |
| 2nd day | 0.067±0.141## | 2.852±0.294** | 1.519±0.412**## | 1.633±0.508**## | 1.867±0.689**## | 2.167±0.236**## |
| 3rd day | 0.100±0.316## | 2.926±0.278** | 1.481±0.380** | 1.833±0.527**## | 1.889±0.624**## | 2.233±0.225**## |
| 4th day | 0.000±0.000## | 3.037±0.309** | 1.333±0.333**## | 1.467±0.450**## | 1.741±0.521**## | 2.067±0.516**## |
| 5th day | 0.000±0.000## | 3.111±0.333** | 1.111±0.333## | 1.300±0.331**## | 1.444±0.373**## | 1.767±0.316**# |
| 6th day | 0.000±0.000## | 3.037±0.351** | 1.037±0.261**## | 1.033±0.246**## | 1.371±0.389**## | 1.533±0.233**## |
| 7th day | 0.000±0.000## | 2.815±0.503** | 1.000±0.236**## | 0.967±0.246**## | 1.148±0.294**## | 1.467±0.172**## |
| 8th day | 0.000±0.000## | 2.444±0.601** | 0.963±0.309**## | 0.933±0.211**## | 1.111±0.289**## | 1.333±0.157**## |
| 9th day | 0.000±0.000## | 2.296±0.539** | 0.889±0.289**## | 0.867±0.172**## | 1.037±0.309**## | 1.267±0.141**## |
| 10th day | 0.000±0.000## | 2.074±0.222** | 0.741±0.324**## | 0.867±0.172**## | 0.926±0.401**## | 1.200±0.172**## |
| 11th day | 0.000±0.000## | 1.926±0.324** | 0.704±0.309**## | 0.733±0.410**## | 0.815±0.338**## | 1.067±0.211**## |
| 12th day | 0.000±0.000## | 1.778±0.236** | 0.667±0.289**## | 0.633±0.367**## | 0.778±0.333**## | 1.033±0.246**## |
| 13th day | 0.000±0.000## | 1.741±0.222** | 0.593±0.278**## | 0.567±0.316**## | 0.667±0.408**## | 0.900±0.161**## |
| 14th day | 0.000±0.000## | 1.704±0.111** | 0.556±0.236**## | 0.481±0.294**## | 0.556±0.333**## | 0.867±0.172**## |
| 15th day | 0.000±0.000## | 1.852±0.556** | 0.481±0.242**## | 0.444±0.289**## | 0.481±0.294**## | 0.833±0.176**## |
| 16th day | 0.000±0.000## | 1.667±0.000** | 0.481±0.242**## | 0.371±0.261**## | 0.407±0.324**## | 0.767±0.161**## |
| 17th day | 0.000±0.000## | 1.778±0.333** | 0.444±0.236**## | 0.259±0.222*## | 0.370±0.309**## | 0.700±0.189**## |
| 18th day | 0.000±0.000## | 1.778±0.333** | 0.333±0.236**## | 0.259±0.222*## | 0.296±0.200*## | 0.600±0.211**## |
| 19th day | 0.000±0.000## | 1.704±0.111** | 0.259±0.147**## | 0.222±0.167**## | 0.296±0.200*## | 0.500±0.176**## |
| 20th day | 0.000±0.000## | 1.704±0.111** | 0.222±0.167*## | 0.222±0.236*## | 0.259±0.222**## | 0.500±0.176**## |
| 21st day | 0.000±0.000## | 1.667±0.000** | 0.433±0.274*## | 0.222±0.236*## | 0.259±0.222**## | 0.400±0.263**## |

Noted: compared with the blank control group, * P<0.05, ** P<0.01; compared with the modeling group, # P<0.05, ## P<0.01. Wherein, "on 0th day" refered to the DAI scores of rats 1 day before the TNBS model had been established.

As shown in table 7 and Fig. 10, in the blank control group, the symptoms of significant body weight loss, stool character abnormal change, e.g. diarrhea, bloody stool and positive stool occult blood had not been found in rats. In the model group, after intracolonical administration of TNBS, the gross blood stool was observed on $1^{st}$ day (namely taking normal saline), which was accompanied with body weight loss. These syptomes were lasted for 6~8 days. On $9^{th}$ day, after administrating normal saline, the stool of rats in this group gradually showed positive stool occult blood, watery stool and gradual body weight increase. DAI scores in the model group were much higher than the one in the blank control group, showing a statistically significant difference ($p < 0.01$). In treatment groups of TCM preparation j of all doses and positive control group, although being treated with tested drugs after intracolonical administration of TNBS, the rats showed body weight loss within previous 6~8 days after administration. Compared with the model group, the body weight in aforethe groups of all doses and positive control group was decreased to a lesser degree. Actually, degree of body weight loss was gradually improved with increase of administration time. Intially, the stool character mainly included positive stool occult blood with no gross blood stool. Until $21^{st}$ day after administration, the stool character in most of rats was recovered except slightly positive stool occult blood in few rats. Wherein, the TCM preparation **J** high-dose group had the most obvious improving effect. There were much lower DAI scores in the TCM preparation **J** group of all doses and the positive control group than the one in model group, having stastically significant difference ($p < 0.01$).

Compared with the TCM preparation **j** group administered intragastrically, the intracolonical administration of high-dose preparation **j** could clearly improve DAI scores in rats.

(2) Corresponding results of serum indice

Table 8 effect of TCM preparation **j** by intracolonical administration on serum content of IFN-γ, IL-1, IL-4, IL-10 and Gln in instestinal mucosa in TNBS model rats (Mean±SD)

| Groups | Dose | IFN-γ (pg/ml) | IL-1 (pg/ml) | IL-4 (pg/ml) | IL-10 (pg/ml) | Gln (μmol/L) |
|---|---|---|---|---|---|---|
| Blank control group | | 76.34±15.00## | 118.19±8.10## | 86.48±14.96## | 97.28±12.19# | 163.67±19.93## |
| Model group | | 111.30±30.32** | 205.13±38.14** | 58.76±8.31** | 80.06±13.44* | 112.00±16.64** |
| Positive control group | 100mg/kg | 84.51±19.65# | 167.66±26.95** # | 96.80±27.51## | 88.06±10.47 | 146.36±28.11# |
| High dose group | 2.023g/kg | 79.37±10.05## | 139.81±17.17## | 76.82±12.30 | 97.75±11.10# | 156.15±26.00## |
| Middle dose group | 1.012g/kg | 97.13±18.95 | 179.90±25.41** | 82.31±20.66# | 85.09±10.72 | 144.83±19.52# |
| Low dose group | 0.506g/kg | 97.40±10.58 | 185.37±43.31** | 63.34±7.86* | 97.45±17.04# | 149.35±23.35## |

Noted: compared with the blank control group, * $P<0.05$, ** $P<0.01$; compared with the model group, # $P<0.05$, ## $P<0.01$.

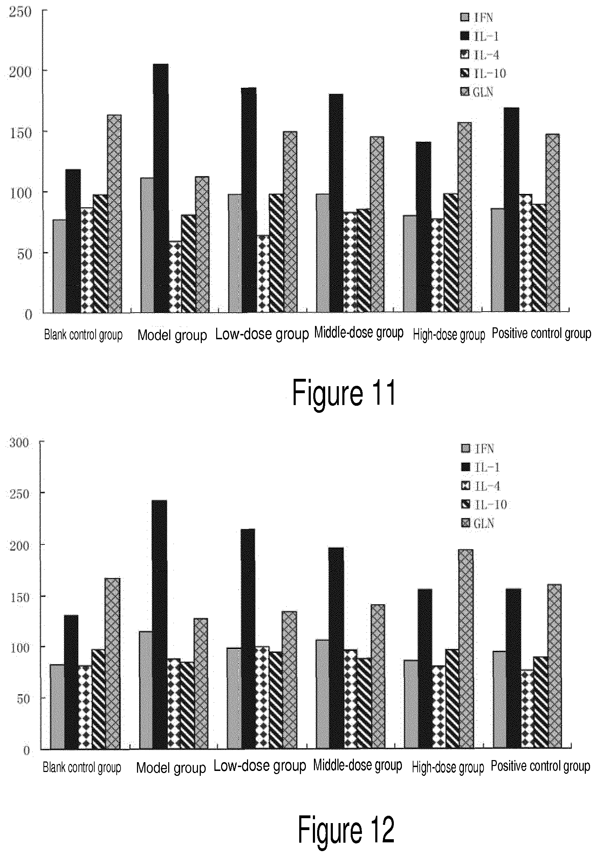

As shown in table 8 and Fig.11, compared with the blank control group, the serum content of IFN-γ was increased significantly in the model group rats, having statistically significant difference (p<0.01). Compared the model group, the serum content of IFN-γ was decreased significantly in the positive control group rats, having statistically significant difference (p<0.01). Compared the model group, the serum content of IFN-γ was decreased to a certain extent in the TCM preparations **j** high-dose, middle-dose and low-dose groups; especially the decreased degree in the rats of the high-dose group was most obvious, having statistically significant difference (p<0.01).

Compared with the blank control group, the serum content of IL-1 was increased markedly in the model group, having statistically significant difference (p<0.01). Compared the model group, the serum content of IL-1 was decreased clearly in the positive control group rats, having statistically significant difference (p<0.01). Compared the model group, the serum content of IL-1 was decreased to a certain extent in the TCM preparations j high-dose, middle-dose and low-dose groups; especially the decreased degree in the rats of the high-dose group was most obvious (p<0.01).

Compared with the blank control group, the mucosa Gln content was decreased markedly in the model group, having statistically significant difference (p<0.01). Compared the model group, the mucosa Gln content was all increased in the TCM preparations **j** high-dose, middle-dose, low-dose groups and model group, having statistically significant difference (respectively p<0.01, p<0.05, p<0.01, p<0.05).

Compared with the blank control group, the serum content of IL-4 was decreased markedly in the model group, having statistically significant difference (p<0.01). Compared the model group, the serum content of IL-4 was increased clearly in the positive control group rats, having statistically significant difference (p<0.01). Compared the model group, the serum content of IL-4 was decreased to a certain extent in the TCM preparations **j** high-dose, middle-dose and low-dose groups; especially the increased degree in the rats of the middle-dose group was most obvious, having statistically significant difference (p<0.05).

Compared with the blank control group, the serum content of IL-10 was decreased slightly in the model group, having statistically significant difference (p<0.05). Compared the model group, the serum content of IL-10 was increased to a certain extent in the rats of positive control group, but did not have statistically significant difference (p<0.01). Compared the model group, the serum content of IL-10 was decreased slightly in the TCM preparations **j** high-dose and middle-dose, having statistically significant difference (p<0.05).

TNBS-induced Crohn's disease is mainly Th1 cell-mediated, displaying the increased content of IFN-γ and IL-1, decreased or normal expression of IL-4 and IL-10. Decreased content of IFN-γ and IL-1 in rats of the TCM preparations **j** group at all doses showed that the TCM preparation had a certain therapeutic effect on TNBS-induced Crohn's disease. Mocusa Gln content in the model group was significantly lower than the one in the blank control group, indicating that TNBS could result in mocusa cell injuries. The TCM preparation **j** was able to increase musoca Gln content in rats of model group, showing that the TCM preparation could be used for treating TNBS-induced Crohn's disease.

Table 9 effect of TCM preparation **j** by intragastric administration on serum content of IFN-γ, IL-1, IL-4, IL-10 and Gln in instestinal mucosa in TNBS model rats (Mean±SD)

| Groups | Dose | IFN-γ (pg/ml) | IL-1 (pg/ml) | IL-4 (pg/ml) | IL-10 (pg/ml) | Gln (μmol/L) |
|---|---|---|---|---|---|---|
| Blank control group | | 82.33±25.07## | 129.85±24.35## | 80.66±20.42 | 96.71±7.69 | 166.54±25.57# |
| Model group | | 114.47±19.28** | 241.62±61.00** | 87.66±14.00 | 83.82±14.19* | 126.92±20.27* |
| Positive control group | 100mg/ kg | 93.88±23.67 | 154.59±39.98## | 75.80±17.47 | 88.56±16.77 | 158.74±41.35 |
| High dose group | 2.023g/ kg | 85.56±6.95# | 155.07±46.51## | 79.95±8.33 | 95.76±11.25 | 193.20±31.59## |
| Middle dose group | 1.012g/ kg | 105.51±24.42* | 195.16±12.11* | 95.74±8.06 | 87.72±6.29 | 139.97±7.55 |
| Low dose group | 0.506g/ kg | 98.64±5.49 | 213.34±48.92** | 99.17±17.52* | 94.00±11.25 | 132.95±33.73 |

Noted: compared with the blank control group, * P<0.05, ** P<0.01; compared with the model group, # P<0.05, ## P<0.01.

As shown in table 9 and Fig.12, compared with the blank control group, the serum content of IFN-$\gamma$ was increased significantly in the model group rats, having statistically significant difference (p<0.01). Compared the model group, the serum content of IFN-$\gamma$ was decreased to a certain extent in the rats of positive control group, and did not have statistically significant difference. Compared the model group, the serum content of IFN-$\gamma$ was decreased to a certain extent in the TCM formulatios j high-dose, middle-dose and low-dose groups; especially the decreased degree in the rats of the high-dose group was most obvious, having statistically significant difference (p<0.05).

Compared with the blank control group, the serum content of IL-1 was increased markedly in the model group, having statistically significant difference (p<0.01). Compared the model group, the serum content of IL-1 was decreased clearly in the rats of positive control group, having statistically significant difference (p<0.01). Compared the model group, the serum content of IL-1 was decreased to a certain extent in the TCM preparations **j** high-dose, middle-dose and low-dose groups; especially the decreased degree in the rats of the high-dose group was most obvious (p<0.01).

Compared with the blank control group, the mucosa Gln content was decreased markedly in the model group, having statistically significant difference (p<0.01). Compared the model group, the mucosa Gln content was all increased in the TCM preparations **j** high-dose, middle-dose, low-dose groups, of which the high-dose group had statistically significant difference (p<0.01).

Compared with the blank control group, the serum content of IL-4 was increased slightly in the model group, and did not have statistically significant difference. Compared the model group, there was not statistically significant difference in serum IL-4 content among the TCM preparations **j** high-dose, middle-dose and low-dose groups and the positive control group.

Compared with the blank control group, the serum content of IL-10 was decreased slightly in the model group, having statistically significant difference (p<0.05). Compared the model group, there was not statistically significant difference in serum IL-10 content among the TCM preparations **j** high-dose, middle-dose and low-dose groups and the positive control group

Compared with the TCM preparation **j** by intragastric administration, serum contents of IFN-$\gamma$, IL-1 and Gln were improved more obviously in the high-dose TCM preparation **j** by intracolonical administration, which was consistent with the result obtained in the DAI scores.

(3) Corresponding results of pathological indice

Table 10 effect of TCM preparation **j** by intracolonical administration on histopathology in instestinal mucosa in TNBS model rats (Mean$\pm$SD)

| Groups | Dose | Gross damage score | Histopathological score |
|---|---|---|---|
| Blank control group | | 0.000$\pm$0.000## | 0.17$\pm$0.41# |
| Model group | | 2.714$\pm$2.289** | 2.83$\pm$2.14* |
| Positive control group | 100mg/kg | 0.500$\pm$0.707## | 1.00$\pm$1.10 |
| High-dose group | 2.023g/kg | 0.200$\pm$0.422## | 0.67$\pm$1.03# |
| Middle-dose group | 1.012g/kg | 0.333$\pm$0.500## | 0.50$\pm$0.55# |
| Low-dose group | 0.506g/kg | 0.875$\pm$1.356## | 1.00$\pm$1.55 |

Noted: compared with the blank control group, * P<0.05, ** P<0.01; compared with the model group, # P<0.05, ## P<0.01.

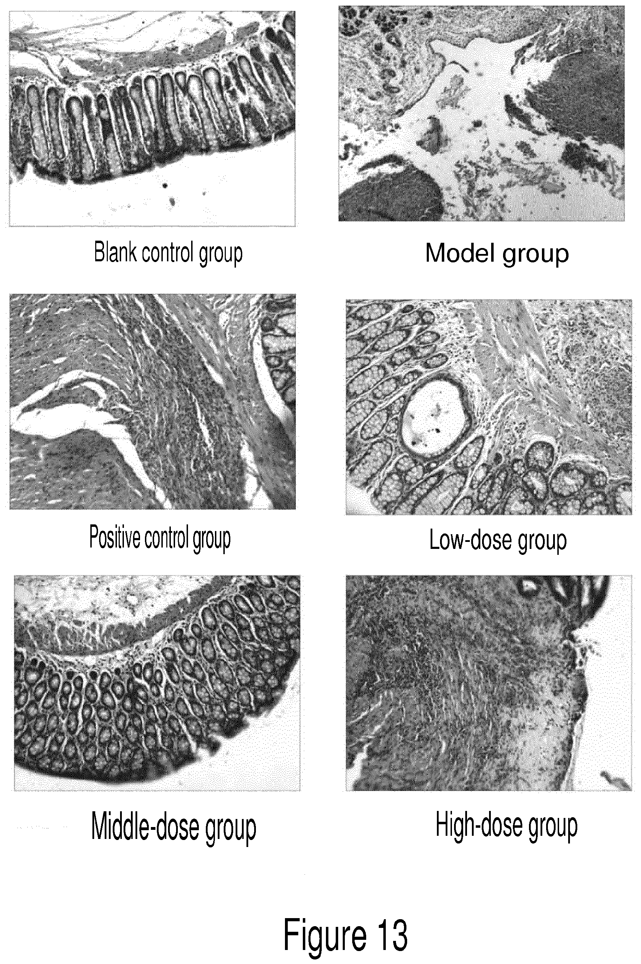

As shown in table 10 and Fig.13, in the blank control group, the intestinal mucosa of rats was smooth with no clear lesion. The mucosa tissue was observed intact by microscope with no congestion and edema, with no inflammatory infiltration and ulcer. In the model group, a serie of symptoms were found in mucosa, including obvious congestion and hematoma, severely thickened bowel wall and intestinal adhersion with peripheral intestine in most of rats. Bowel dilation, pneumatosis and multiple ulcers occurred in part of rats, some of which even developed into large and deep ulcers. Under microscope, inflammation or ulcer had been observed, some of which showed the transmural inflammation and the ulcer invaded serous layer and perforated. In the rats of positive control group, except a small number of rats with mild congestion edema in intestinal tissue, significant lesions were not found in most of rats. Under the microscope, except 1 case having visible lesion, there was no abnormal change in most of rats. A small amount of rats were found to have slight congestion edema in bowel wall in the TCM preparation **j** high-dose, middle-dose and low-dose groups. Compared with the model group, colon tissue lesions were all improved; especially the decreased degree in rats of the high-dose and middle-dose groups was more obvious. Mucosa gross damage score and histopathological score were obviously higher than normal value in comparison with the rats in blank control group, havng statistically significant difference (p<0.01). Mucosa gross damage score and hispatholoigical score were obviously decreased in the TCM formulatios **j** high-dose, middle-dose and low-dose groups; especially the decreased degree of both scores in the rats of the high-dose group was most obvious, having statistically significant difference (respectively p<0.01 and p<0.05).

Table 11 effect of TCM preparation **j** by intragastrical administration on histopathology in instestinal mucosa in TNBS model rats (Mean±SD)

| Groups | Dose | Gross damage score | Histopathological score |
|---|---|---|---|
| Blank control group | | 0.000±0.000## | 0.00±0.00## |
| Model group | | 3.444±0.527** | 2.50±1.76** |
| Positive control group | 100mg/kg | 0.556±0.527*## | 0.83±0.41**# |
| High-dose group | 2.023g/kg | 0.222±0.441## | 0.17±0.41# |
| Middle-dose group | 1.012g/kg | 0.333±0.500## | 0.17±0.41# |
| Low-dose group | 0.506g/kg | 0.600±0.516**## | 0.50±0.55*# |

Noted: compared with the blank control group, * P<0.05, ** P<0.01; compared with the model group, # P<0.05, ## P<0.01.

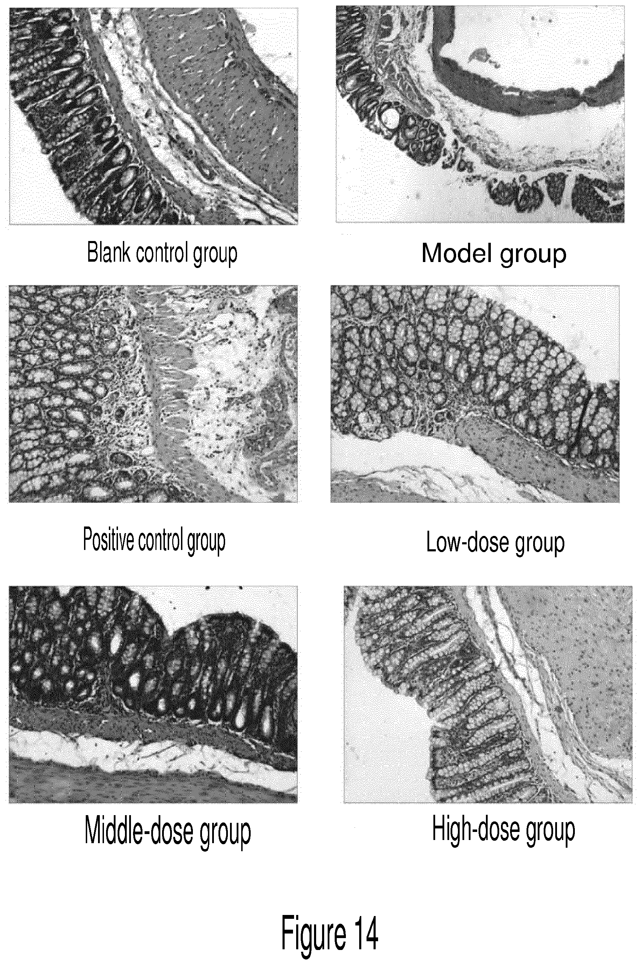

As shown in table 11 and Fig.14, in the rats of blank control group, the intestinal mucosa was smooth with no obvious lesion. The mucosa tissue was observed intact by microscope with no congestion and edema, with no inflammatory infiltration and ulcer. In the model group, a serie of symptoms were found in mucosa, including obvious congestion and hematoma, severely thickened bowel wall and tissue adhersion around intestine in most of rats. A part of rats had bowel dilation, pneumatosis and multiple ulcers and large and deep ulcers were formed partially. Under microscope, inflammation or ulcer had been observed, a part of which showed the transmural inflammation and the ulcer invaded serous layer and perforated. In the rats of positive control group, except a small number of rats with mild conjection edema in intestinal tissue, significant lesions were not found in most of rats. Under the microscope, compared with the model group, colon tissue lesion was alleviated; especially the decreased degree in rats of the high-dose and middle-dose groups was most obvious. Mucosa gross damage score and hispatholoigical score were obviously higher than normal value, compared with the rats in blank control group, having statistically significant difference (p<0.01). Mucosa gross damage score and hispatholoigical score were obviously decreased in the TCM formulatios j high-dose, middle-dose and low-dose groups; especially both scores were decreased most obviously in the rats of the high-dose group, having statistically significant difference (respectively p<0.01 and p<0.05).

As shown in the research, both intragastric and intracolonical administration of the TCM preparation **j** was able to alleviate the symptoms of TNBS-induced Crohn's disease in rats, reduce increased amplitude of serum content of INF-γ and IL-1 and increase mucosa Gln content.

**Trial example 6** prevention and treatment effect of the TCM preparation **k** on oxazolone-caused Crohn's disease rat model

1. Aminals: SD rats, half male and female, weighing 180~220g, were purchased from Aminal center, Institute of Hygiene and Environmental Medicine, Academy of Military Medical Sciences of PLA with the certificate Number SCXK-2009-003.

2. Drug and reagent: mesalamine, used as positive drug, was provided by Tasly Pharmaceutical Group Co. Ltd. 5% OXZ was purchased from Sigma Inc. IFN-γ, IL-4 and Gln Elisa assay kits were purchased from bioswamp Inc. Stool occult blood assay kit was provided by Nanjing Jiancheng Bioengineering Institute.

3. Apparatus: AB135-S electronic analytical scale was purchased from Mettle Toledo Inc. Switzerland.

Method:

Grouping: the experiment was carried out by using two ways of intracolonical and intragastric administration. Hence, rats were divided into following groups: the blank control intragastrical group, the model intragastrical group the positive control intragastrical group, the TCM preparation **k** high-dose intragastrical group, the middle-dose intragastrical group, the low-dose intragastrical group, the blank control intracolonical group, the model intracolonical group, the positive control intracolonical group, the TCM preparation **k** high-dose intracolonical group, the middle-dose intracolonical group and the low-dose intracolonical group. There were 10 rats each group.

Method for establishing rat model: referring to the Heller method, OXZ was used to induce the SD rat Crohn's disease model (abbreviated as "OXZ model"). Neck and back skin of rats were shaved (2cm×2cm). Ethanol was used to prepare 5% (w/v) OXZ solution, and 0.3ml resultant OXZ solution was dripped on the skin of rats. After the solution was air-dried naturally, skin allergy occurred in rats. The sensitized rats were adaptively raised for 5 days, clysterd and anesthetized by intraperitoneal injection of pentobarbital sodium. A latex tube with outer diameter of 2mm was inserted the anus 6~8 cm inside, through which 0.45ml of 5% OXZ solution (w/v) was injected into the anus; the solution was prepared with 50% (v/v) ethanol. After completion, the rats were inverted by lifting tails for 60s to make the solution totally absorbed. Rats in the blank control group were treated by the same method with 50% (v/v) ethanol with no OXZ. If the rats manifested dark hair color, body weight loss, poor appetite, lassitude, watery stool or diarrhea, the model establishment was success.

Administration method: after successful model establishment by OXZ, the drug was given to rats. The TCM preparation **k** saline solutions at corresponding dose were administered to the groups of preparation **k** of all doses and the mesalamin saline solution, a positive drug, administrated to the positive control group. The rats of blank control group were normally fed, given with water and the same volume of normal saline was given in the same administration routes by referring to the dosage regime of aforesaid treatment groups. The rats of model group were administrated with the same volume of normal saline in the same administration routes. The dose of each group was as follows: mesalamin group at 100mg/kg rat weight, the TCM preparation **k** high-dose group at 2.023g raw medicine/kg rat weight, the middle-dose group at 1.012g raw medicine/kg rat weight and the low-dose group at 0.506g raw medicine/kg rat weight. In both intragastric and intracolonical routes, test drugs saline solution or normal saline were administrated in a ratio of 0.7ml/100g rat weight, once per day for 11 days consecutively. At $5^{th}$ day after administration, 5% OXZ solution was given by the modeling method to the TCM preparation **k** high-dose, middle-dose and low-dose groups, the positive control group, the model group to establish model, and 50% ethanol was intrarectally given to the blank control group.

After being treated as disclosed above, the rats in each group were evaluated by following indice.

(1) General Indice: weight change and stool (e.g. stool occult blood, bloody stool and diarrhea) were observed everyday to calculate DAI score.

$$DAI\ score=(weight\ loss\ percentage+defecate\ character\ fraction+\ hematochezia\ fraction)/3$$

Table 12 DAI score criteria

| Weight loss percentage (wt%) | Stool character | Stool occult blood/gross blood stool | Score |
| --- | --- | --- | --- |
| 0% | Normal | Normal | 0 |
| 1%~5% | Slightly loose | Positive stool occult blood | 1 |
| 5%~10% | Loose | Increased positive stool occult blood | 2 |
| 10%~15% | Slightly watery stool | Gross blood stool | 3 |
| >15% | Watery stool | Increased gross blood stool | 4 |

(2) Serum indice: after final administration (on $11^{th}$ day), the rats were fasted but given water. 24 hours later, blood was gained from abdominal arota. Serum IFN-$\gamma$, IL-4, and Gln in intestinal musoca were determined by ELISA in rats.

(3) Phathological indice: the colon of rats was taken out. The gross colon tissue damage was scored, followed by histopathological examination and scoring by conventional HE staining. The scoring criteria were seen in table 13.

Table 13 colon tissue damage and histopathological scoring criteria

| Score | Gross damage scoring criteria | Score | Histopathological scoring criteria |
| --- | --- | --- | --- |
| 0 | No damage | 0 | Normal colon tissue |
| 1 | Congestion and edema occurs but no ulcer | 1 | Inflammation or ulcer site occurs |
| 2 | Ulcer occurs but no significant inflammation | 2 | Inflammation or ulcer sites are restricted to mocusa and submocusa |

(continued)

| Score | Gross damage scoring criteria | Score | Histopathological scoring criteria |
|---|---|---|---|
| 3 | Ulcer occurs but just one inflammation | 3 | Inflammation or ulcer sites invade muscularis propria |
| 4 | More than 2 ulcers and inflammations occurs and ulcer stie<1cm | 4 | Transmural inflammation or ulcer sites invade serous layer |
| 5 | More than 2 ulcers and inflammations occurs, at leat one ulcer or inflammation site > 1cm | 5 | Transmural inflammation or large area of ulcer invades serous layer and perforates |

Statistical analysis: Spss 17.0 statistical software was used to analyze above scores. Data were expressed as Mean+SD and t-test used for comparison between groups. P≤0.05 showed a statistically significant difference.

By intragastric administration or intracolonical administration, the results were seen following table 14~19.

(1) Results of general indice (DAI scores)

Table 14 effect of TCM preparation **k** by intracolonical administration on DAI scores in OXZ model rats (Mean±SD)

| Administration days | Blank control group | Modeling group | Positive control group (100mg/kg) | High-dose (2.023g/kg) | Middle-dose (1.012g/kg) | Low-dose (0.506g/kg) |
|---|---|---|---|---|---|---|
| 1st day | 0.100±0.1613## | 0.467±0.172** | 0.267±0.263# | 0.333±0.272* | 0.233±0.225# | 0.337±0.105** |
| 2nd day | 0.033±0.105# | 0.300±0.189* | 0.400±0.378** | 0.482±0.338** | 0.367±0.189** | 0.333±0.272* |
| 3rd day | 0.000±0.000 | 0.067±0.141 | 0.300±0.331* | 0.481±0.412**## | 0.400±0.306**# | 0.233±0.316 |
| 4th day | 0.000±0.000 | 0.133±0.172 | 0.333±0.385* | 0.370±0.389** | 0.370±0.351** | 0.200±0.281 |
| 5th day | 0.000±0.000## | 0.567±0.225** | 0.800±0.422** | 0.815±0.338** | 0.778±0.167** | 0.630±0.351** |
| 6th day | 0.000±0.000## | 1.967±0.761** | 1.833±0.550** | 1.815±0.818** | 1.667±0.645** | 1.704±0.889** |
| 7th day | 0.067±0.141## | 2.333±0.588** | 1.700±0.637** | 1.593±0.830**# | 1.259±0.778**## | 1.370±1.006**## |
| 8th day | 0.033±0.105## | 2.667±0.77** | 1.500±0.593**## | 1.296±0.772**## | 0.875±0.533**## | 0.926±0.760**## |
| 9th day | 0.067±0.141## | 2.833±0.527** | 0.733±0.439**## | 0.889±0.645**## | 0.571±0.317*## | 0.815±0.669**## |
| 10th day | 0.000±0.000## | 2.733±0.783** | 0.700±0.576**## | 0.583±0.636*## | 0.333±0.272## | 0.407±0.364## |
| 11th day | 0.000±0.000## | 2.852±0.603** | 0.433±0.649*## | 0.250±0.236## | 0.143±0.262## | 0.370±0.389## |

Noted: compared with the blank control group, * P<0.05, ** P<0.01; compared with the model group, # P<0.05, ## P<0.01.

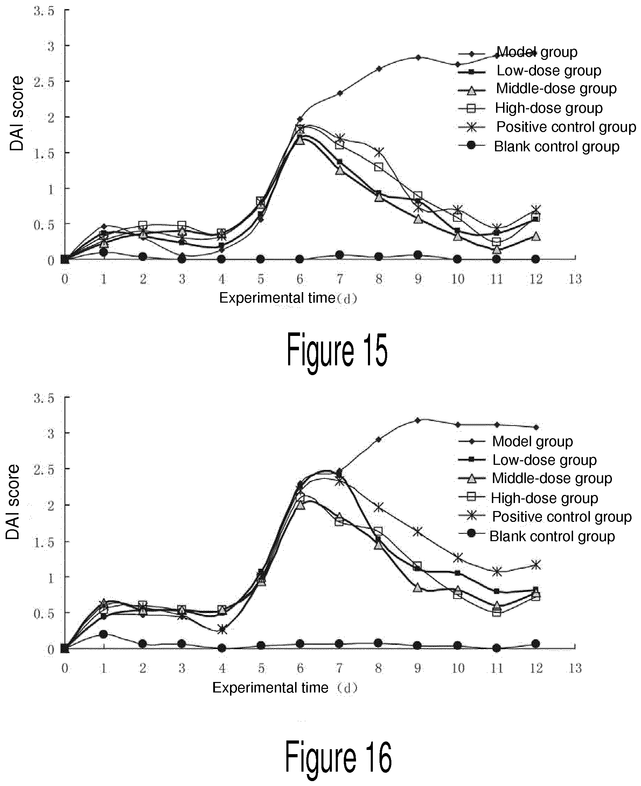

As shown in table 14 and Fig.15, in the blank control group, the symptoms of significant body weight loss, stool character abnormal change, e.g. diarrhea, bloody stool and positive stool occult blood had not been found in rats. In rats of the OXZ model group with skin allergy, after the rats were given normal saline (namely on 1st day), the body weight dropped slightly, but no significant change had been found on stool characters. However, after intracolonical administration of OXZ (5th day after administration of normal saline), the stool of rats in the group gradually showed positive stool occult blood, watery stool which was accompanied with body weight loss. DAI scores in the model group were much higher than that in the blank control group, showing a statistically significant difference. In TCM preparation **k** groups of all doses and positive control group, after skin sensitization with the OXZ, on 1st day after administration, the body weight dropped slightly. Within previous 1~5 days after administration, no significant change had been found on stool characters. After intracolonical administration of OZX (5th day after administration), no significant body weight loss was found, compared with the model group, having a statistically significant difference. In TCM preparation **k** groups of all doses and positive control group, after intracolonical administration of OZX, initially, the stool characters mainly showed positive stool occult blood with no significant gross blood stool. Until 11th day after administration, the stool character in a part of rats showed positive stool occult blood with no significant watery stool and the stool character in most of rats was recovered. There was significant improving effect in TCM preparation **k** high-dose and middle-dose groups. The DAI scores in the TCM preparation **k** group of all doses were significantly lower than the one in model group, having stastically significant difference (p<0.01).

Table 15 effect of TCM preparation **k** by intracolonical administration on DAI scores in TNBS model rats (Mean±SD)

| Administration days | Blank control group | Modeling group | Positive control group (100mg/kg) | High-dose (2.023g/kg) | Middle-dose (1.012g/kg) | Low-dose (0.506g/kg) |
|---|---|---|---|---|---|---|
| 1st day | 0.200±0.172# | 0.433±0.274* | 0.600±0.141** | 0.533±0.172** | 0.633±0.189**# | 0.433±0.274* |
| 2nd day | 0.067±0.141# | 0.467±0.233** | 0.567±0.225** | 0.600±0.211** | 0.533±0.172** | 0.533±0.233** |
| 3rd day | 0.067±0.141# | 0.433±0.225** | 0.467±0.233** | 0.533±0.281** | 0.533±0.172** | 0.519±0.242** |
| 4th day | 0.000±0.000# | 0.267±0.263* | 0.267±0.211* | 0.533±0.233**# | 0.533±0.233**# | 0.481±0.294**# |
| 5th day | 0.033±0.105## | 0.967±0.292** | 1.000±0.222** | 0.967±0.189** | 0.933±0.306** | 1.074±0.222** |
| 6th day | 0.067±0.141## | 2.300±0.637** | 2.200±0.322** | 2.100±0.754** | 2.000±0.889** | 2.259±0.813** |
| 7th day | 0.067±0.141## | 2.467±0.834** | 2.333±0.351** | 1.767±0.876**# | 1.833±0.920** | 2.407±0.830** |
| 8th day | 0.074±0.147## | 2.900±0.498** | 1.967±0.576**## | 1.633±0.949**## | 1.444±1.067**## | 1.519±0.852**## |
| 9th day | 0.037±0.111## | 3.167±0.283** | 1.633±0.429**## | 1.148±0.930**## | 0.852±0.556**## | 1.111±0.986**## |
| 10th day | 0.037±0.111## | 3.111±0.471** | 1.267±0.625**## | 0.741±0.795*## | 0.815±0.669*## | 1.042±0.983**## |
| 11th day | 0.000±0.000## | 3.111±0.471** | 1.067±0.798**## | 0.500±0.591## | 0.593±0.662## | 0.792±1.097*## |

Noted: compared with the blank control group, * P<0.05, ** P<0.01; compared with the modeling group, # P<0.05, ## P<0.01.

EP 3 015 111 B1

As shown in table 15 and Fig.16, in the blank control group, the symptoms of significant body weight loss, stool character abnormal change, e.g. diarrhea, bloody stool and positive stool occult blood had not been found in rats. In the rats of OXZ model group with skin allergy, after the rats were given normal saline (namely on 1st day), the body weight dropped slightly, but no significant change had been found on stool characters. However, after intracolonical administration of OXZ (5th day after administration of normal saline), the stool of rats in this group gradually showed positive stool occult blood, watery stool which was accompanied with body weight loss.DAI scores in the model group were much higher than the one in the blank control group, showing a statistically significant difference. In TCM preparation **k** groups of all doses and positive control group, after skin sensitization with the OXZ, on 1st day after administration, the body weight dropped slightly. Within previous 1~5 days after administration, no significant change had been found on stool characters. After intracolonical administration of OZX (5th day after administration), no significant body weight loss was found, compared with the model group, having a statistically significant difference. In TCM preparation **k** groups of all doses and positive control group, after intracolonical administration of OZX, initially, the stool characters mainly showed positive stool occult blood with no significant gross blood stool. Until 11th day after administration, the stool character in a part of rats showed positive stool occult blood with no significant watery stool and the stool character in most of rats was recovered. There was significant improving effect in TCM preparation k high-dose and middle-dose groups. The DAI scores in the TCM preparation **k** group of all doses were significantly lower than the one in model group, having stastically significant difference (p<0.01).

Compared with the TCM preparation **k** intracolonical group, intragastric administration of high-dose and middle-dose preparations **k** had stronger effect on improving DAI scores in rats.

(2) Corresponding results of serum indice

Table 16 effect of TCM preparation k by intracolonical administration on serum content of IFN-$\gamma$, IL-4 and Gln in instestinal mucosa in OXZ model rats (Mean $\pm$ SD)

| Groups | Dose | IFN-$\gamma$ (pg/ml) | IL-4(pg/ml) | Gln ($\mu$mol/L) |
|---|---|---|---|---|
| Blank control group | | 80.54$\pm$9.34 | 82.02$\pm$11.22## | 152.92$\pm$17.29## |
| Model group | | 82.40$\pm$6.35 | 108.97$\pm$21.45** | 104.66$\pm$15.13** |
| Positive control group | 100mg/kg | 85.85$\pm$10.66 | 86.63$\pm$11.20# | 124.06$\pm$24.40* |
| High dose group | 2.023g/kg | 79.39$\pm$10.60 | 80.25$\pm$11.15## | 133.16$\pm$8.70# |
| Middle dose group | 1.012g/kg | 87.96$\pm$7.09 | 89.68$\pm$13.28# | 109.74$\pm$25.34** |
| Low dose group | 0.506g/kg | 84.89$\pm$9.69 | 93.26$\pm$16.71 | 106.23$\pm$23.74** |

Noted: compared with the blank control group, * P<0.05, ** P<0.01; compared with the model group, # P<0.05, ## P<0.01.

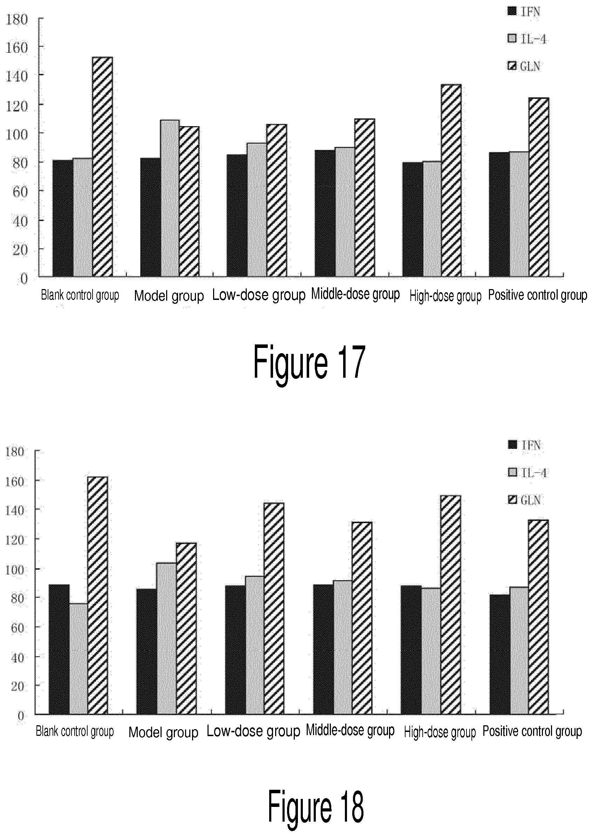

As shown in table 16 and Fig.17, compared with the blank control group, no significant difference had been found on the serum content of IFN-$\gamma$ in rats. Compared the model group, no significant difference had been found on the serum content of IFN-$\gamma$ in the TCM formulatios k high-dose, middle-dose and low-dose groups and positive control group.

Compared with the blank control group, the serum content of IL-4 was increased markedly in the model group, having statistically significant difference (p<0.01). Compared the model group, the serum content of IL-4 was decreased clearly in the positive control group rats, having statistically significant difference (p<0.05). Compared the model group, the serum content of IL-4 was decreased to a certain extent in the TCM preparations k high-dose and middle-dose groups, having statistically significant difference (respectively p<0.01 and p<0.05).

Compared with the blank control group, the mucosa Gln content was decreased markedly in the model group, having statistically significant difference (p<0.01). Compared the model group, the mucosa Gln content was all increased in the TCM preparations k high-dose, middle-dose, low-dose groups and model group, having statistically significant difference (respectively p<0.01, p<0.01 and p<0.05).

OXZ-induced Crohn's disease is mainly Th2 cell-mediated, displaying the increased content of IL-4, decreased or normal expression of IFN-γ. The TCM preparations k group at all doses could decrease the content of IL-4 in rats, showing that the TCM preparation had a certain thereaputic effect on OXZ-induced Crohn's disease. Mocusa Gln content in the model group was significantly lower than the one in the blank control group, indicating that OXZ could result in mocusa cell injuries. The TCM preparation k was able to increase musoca Gln content in rats of model group, showing that the TCM preparation could be used for treating OXZ-induced Crohn's disease.

Table 17 effect of TCM preparation k by intragastric administration on serum content of IFN-γ, IL-4 and Gln in instestinal mucosa in OXZ model rats (Mean ± SD)

| Groups | Dose | IFN-γ (pg/ml) | IL-4 (pg/ml) | Gln (μmol/L) |
|---|---|---|---|---|
| Blank control group | | 88.27±17.83 | 76.04±10.1## | 161.72±28.23# |
| Model group | | 85.33±19.83 | 103.24±10.51** | 116.34±31.83* |
| Positive control group | 100mg/kg | 81.88±18.00 | 86.86±7.84# | 132.59±41.74 |
| High dose group | 2.023g/kg | 87.85±15.43 | 86.04±14.65# | 149.37±12.70 |
| Middle dose group | 1.012g/kg | 88.55±20.87 | 91.75±7.99# | 131.32±26.48 |
| Low dose group | 0.506g/kg | 87.58±20.64 | 94.76±7.45** | 144.34±40.90 |

Noted: compared with the blank control group, * P<0.05, ** P<0.01; compared with the model group, # P<0.05, ## P<0.01.

As shown in table 17 and Fig.18, compared with the blank control group, no significant difference was found on the serum content of IFN-γ in the model group rats. Compared the model group, no significant difference was found on the serum content of IFN-γ in the TCM formulatios k high-dose, middle-dose and low-dose groups and positive control group.

Compared with the blank control group, the serum content of IL-4 was increased significantly in the model group, having statistically significant difference (p<0.01). Compared the model group, the serum content of IL-4 was decreased significantly in the positive control group, having statistically significant difference (p<0.05). Compared the model group, the serum content of IL-4 was decreased to a certain extent in the TCM preparations k high-dose, middle-dose and low-dose groups; especially having statistically significant difference in the rats of the high-dose and middle-dose groups (respectively p<0.05 and p<0.05).

Compared with the blank control group, the mucosa Gln content was decreased markedly in the model group, having statistically significant difference (p<0.01). Compared the model group, the mucosa Gln content was all increased to a certain extent in the TCM preparations k high-dose, middle-dose, low-dose groups, having no statistically significant difference.

Compared with the TCM preparation k by intragastric administration, serum content of IL-4 was improved more obviously in the high-dose TCM preparation k by intracolonical administration.

(3) Corresponding results of pathological indice

Table 18 effect of TCM preparation k on mucosa histopathology in rats of OXZ-model(Mean± SD)

| Groups | Dose | Gross damage score | Histopathological score |
|---|---|---|---|
| Blank control group | | 0.000±0.000## | 0.00±0.00## |
| Model group | | 3.556±0.527** | 4.67±0.52** |
| Positive control group | 100mg/kg | 1.800±1.229**## | 3.17±1.33**# |
| High-dose group | 2.023g/kg | 1.571±0.535**## | 2.17±1.47**## |
| Middle-dose group | 1.012g/kg | 1.222±1.302*## | 3.33±1.97** |

(continued)

| Groups | Dose | Gross damage score | Histopathological score |
|---|---|---|---|
| Low-dose group | 0.506g/kg | 2.000±1.528**## | 2.50±1.64**# |

Noted: compared with the blank control group, * P<0.05, ** P<0.01; compared with the model group, # P<0.05, ## P<0.01.

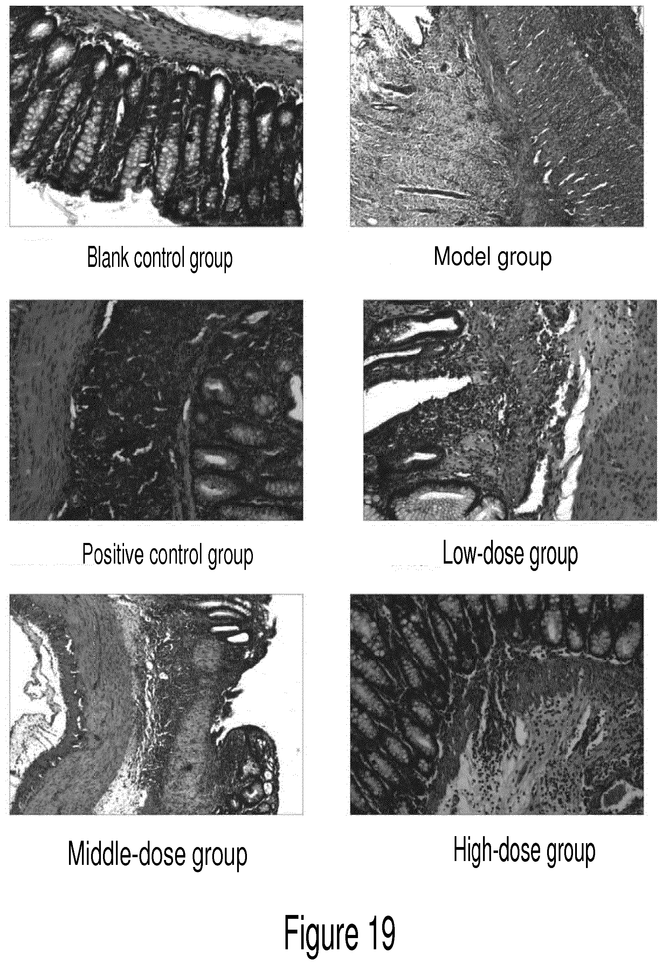

As shown in table 18 and Fig.19, in blank control group, the intestinal mucosa of rats was smooth with no clear lesion. Except inflammation was found in 1 case, no congestion, edema and ulcer were observed in the mucosa tissue by microscope. In the model group, a serie of symptoms occured in mucosa, including obvious congestion and hematoma, severely thickened bowel wall and intestinal adhersion with peripheral tissue in most of rats. Bowel dilation, pneumatosis and multiple ulcers occured in a part of rats, some of which even developed into large and deep ulcers. Under microscope, obvious colon lesion had been observed, and the colon tissue showed the transmural inflammation and the ulcer invaded serous layer and perforated. In the rats of positive control group, inflammation and ulcer were observed in some of intestinal tissue. Compared with the model group, the lesions were released slightly. In the TCM preparation k high-dose, middle-dose and low-dose groups, the colon disease was alleviated somewhat, in which the high-dose group had more obviously improving effect. Mucusa gross damage score and histopathological score were obviously higher than normal value in the model group, having a statistically significant difference (p<0.01) in comparion with the blank control group. Mucusa gross damage score and histopathological score were decreased in the TCM preparation k high-dose, middle-dose and low-dose groups; especially both scores were decreased most obviously in rats of high-dose group, having statistically significant difference (respectively p<0.01 and p<0.01).

Table 19 effect of TCM preparation k by intragastrical administration on histopathology in instestinal mucosa in OXZ model rats (Mean± SD)

| Groups | Dose | Gross damage score | Histopathological score |
|---|---|---|---|
| Blank control group | | 0.000±0.000## | 0.17±0.41## |
| Model group | | 3.667±0.500** | 3.50±1.22** |
| Positive control group | 100mg/kg | 1.700±1.494**## | 2.33±1.21 ** |
| High-dose group | 2.023g/kg | 1.375±0.744**## | 2.83±1.33** |
| Middle-dose group | 1.012g/kg | 1.143±1.215*## | 2.33±1.63** |
| Low-dose group | 0.506g/kg | 2.111±1.364**## | 3.17±1.84** |

Noted: compared with the blank control group, * P<0.05, ** P<0.01; compared with the model group, # P<0.05, ## P<0.01.

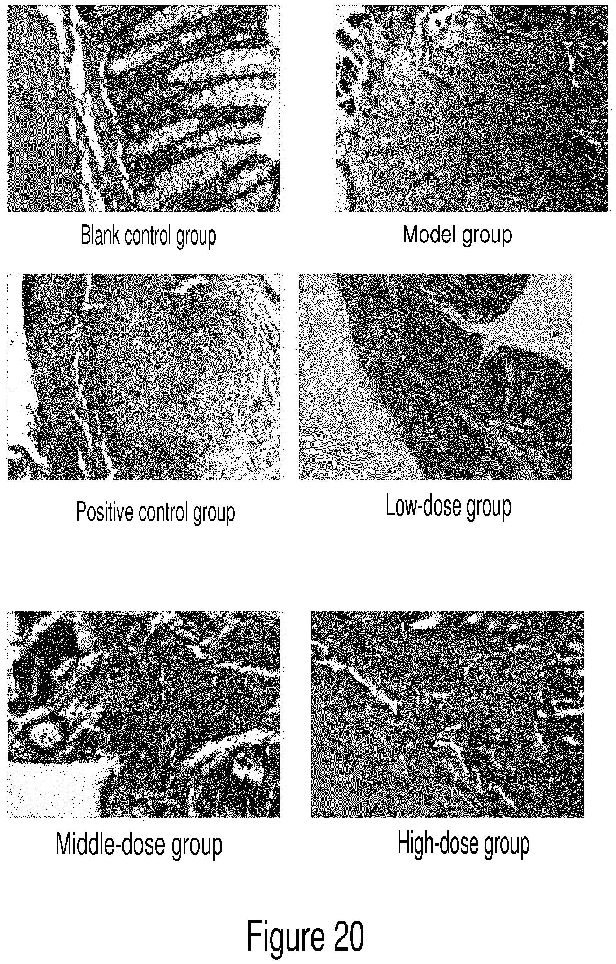

As shown in table 19 and Fig.20, in blank control group, the intestinal mucosa of rats was smooth with no clear lesion. No lesion was observed under microscope. In the model group, a serie of symptoms occured in mucosa, including obvious congestion and hematoma, severely thickened bowel wall and intestinal adhersion with peripheral tissue in most of rats. Bowel dilation, pneumatosis and multiple ulcers occured in the rats, some of which even developed into large and deep ulcers. Under microscope, obvious colon lesion had been observed, and the colon tissue showed the transmural inflammation and the ulcer invaded serous layer and perforated. Compared with the model group, the intestinal lesions were released slightly in the positive control group, but the inflammation or ulcer was still visible and a small number of rats had the transmural inflammation. Compared with the model group, the colon lesion was alleviated somewhat in the TCM preparation high-dose, middle-dose and low-dose groups, in which the high-dose group had more obviously improving effect. Mucusa gross damage score and histopathological score were obviously higher than normal value in the model group, having a statistically significant difference (p<0.01) in comparion with the blank control group. Mucusa gross damage score and histopathological score were decreased in the TCM preparation k high-dose, middle-dose and low-dose groups; especially both scores were decreased most obviously in rats of high-dose group, having statistically significant difference (respectively p<0.01 and p<0.01).

As shown in the research, both intragastric and intracolonical administration of the TCM preparation k were able to alleviate the symptoms of OXZ-induced Crohn's disease in rats, reduce increased amplitude of serum content of IL-4. Intracolonical administration of the TCM preparation k was able to produce a better improving effect than the intragastric.

**EP 3 015 111 B1**

**Claims**

1. Traditional Chinese medicine preparation for use in preventing and/or treating the Crohn's disease, wherein said preparation comprises the raw materials as 1-10 wt % of Sanguis Draxonis, 15-40 wt % of Radix Paeoniae Rubra, 1-20 wt % of Indigo Naturalis, 1-10 wt % of Halloysitum Rubrum, 15-40 wt % of Catechu, 1-10 wt % of Calcined Alumen, 5-30 wt % of Rhizoma Bletillae and 1-10 wt % of Calamina.

2. The traditional Chinese medicine preparation for use of claim 1, wherein said preparation comprises the raw materials as 3-7 wt % of Sanguis Draxonis, 25-35 wt % of Radix Paeoniae Rubra, 5-15 wt % of Indigo Naturalis, 1-5 wt % of Halloysitum Rubrum, 25-35 wt % of Catechu, 1-5 wt % of Calcined Alumen, 10-20 wt % of Rhizoma Bletillae and 1-5 wt % of Calamina.

3. The traditional Chinese medicine preparation for use of claim 1, wherein said preparation comprises the raw materials as 5.1 wt % of Sanguis Draxonis, 30.3 wt % of Radix Paeoniae Rubra, 10.1 wt % of Indigo Naturalis, 3 wt % of Halloysitum Rubrum, 30.3 wt % of Catechu, 3 wt % of Calcined Alumen, 15.2 wt % of Rhizoma Bletillae and 3 wt % of Calamina.

4. The traditional Chinese medicine preparation for use of any one of claims 1 to 3, wherein said preparation includes pharmaceutically acepatable carrier and is prepared into any one of dosage forms: enema; tablet, capsule, oral lquid, buccal tablet, granule, instant powder, pill, dripping pill, pulvis, paste, Dan, suspension, powder, solution, suppository, cream, spray, dry powder inhalation, aerosol, drop, lozenge and patch.

5. Traditional Chinese medicine preparation for use in preventing and/or treating extraintestinal manifestations and complications of the Crohn's disease, wherein said preparation comprises the raw materials as 1-10 wt % of Sanguis Draxonis, 15-40 wt % of Radix Paeoniae Rubra, 1-20 wt % of Indigo Naturalis, 1-10 wt % of Halloysitum Rubrum, 15-40 wt % of Catechu, 1-10 wt % of Calcined Alumen, 5-30 wt % of Rhizoma Bletillae and 1-10 wt % of Calamina.

6. The traditional Chinese medicine preparation for use of claim 5, wherein said preparation comprises the raw materials as 3-7 wt % of Sanguis Draxonis, 25-35 wt % of Radix Paeoniae Rubra, 5-15 wt % of Indigo Naturalis, 1-5 wt % of Halloysitum Rubrum, 25-35 wt % of Catechu, 1-5 wt % of Calcined Alumen, 10-20 wt % of Rhizoma Bletillae and 1-5 wt % of Calamina.

7. The traditional Chinese medicine preparation for use of claim 5, wherein said preparation comprises the raw materials as 5.1 wt % of Sanguis Draxonis, 30.3 wt % of Radix Paeoniae Rubra, 10.1 wt % of Indigo Naturalis, 3 wt % of Halloysitum Rubrum, 30.3 wt % of Catechu, 3 wt % of Calcined Alumen, 15.2 wt % of Rhizoma Bletillae and 3 wt % of Calamina.

8. The traditional Chinese medicine preparation for use of any one of claims 5 to 7, wherein said complications include one or more kinds of intestinal obstruction, intestinal fistula, lower gastrointestinal massive hemorrhage, intestinal perforation, septicemia, abdominal abscess and anal diseases.

9. The traditional Chinese medicine preparation for use of any one of claims 5 to 7, wherein said extraintestinal manifestations include oral mucositis, e.g. the aphthous ulcer; skin pathological changes, e.g. the erythema nodosum, the pyoderma gangrenosum (PG) and non-specific rash; eye pathological changes, e.g. the iridocyclitis and conjunctivitis; arthral lesion, e.g. the arthral pain and arthritis; hepatobiliary lesion, e.g. abnormal liver function and gallstone.

10. The traditional Chinese medicine preparation for use of any one of claims 5 to 7, wherein said preparation includes pharmaceutically acepatable carrier and prepared into any one of dosage forms: enema; tablet, capsule, oral lquid, buccal tablet, granule, instant powder, pill, dripping pill, pulvis, paste, Dan, suspension, powder, solution, suppository, cream, spray, dry powder inhalation, aerosol, drop, lozenge and patch.

**Patentansprüche**

1. Zubereitung der Traditionellen Chinesischen Medizin zur Verwendung beim Vorbeugen und/oder Behandeln von Morbus Crohn, wobei die Zubereitung als Rohmaterialien 1-10 Gew.-% Sanguis Draxonis, 15-40 Gew.-% Radix Paeoniae Rubra, 1-20 Gew.-% Indigo Naturalis, 1-10 Gew.-% Halloysitum Rubrum, 15-40 Gew.-% Katechu, 1-10 Gew.-% kalzinierten Alaun, 5-30 Gew.-% Rhizoma Bletillae und 1-10 Gew.-% Galmei umfasst.

2. Zubereitung der Traditionellen Chinesischen Medizin zur Verwendung nach Anspruch 1, wobei die Zubereitung als Rohmaterialien 3-7 Gew.-% Sanguis Draxonis, 25-35 Gew.-% Radix Paeoniae Rubra, 5-15 Gew.-% Indigo Naturalis, 1-5 Gew.-% Halloysitum Rubrum, 25-35 Gew.-% Katechu, 1-5 Gew.-% kalzinierten Alaun, 10-20 Gew.-% Rhizoma Bletillae und 1-5 Gew.-% Galmei umfasst.

3. Zubereitung der Traditionellen Chinesischen Medizin zur Verwendung nach Anspruch 1, wobei die Zubereitung als Rohmaterialien 5,1 Gew.-% Sanguis Draxonis, 30,3 Gew.-% Radix Paeoniae Rubra, 10,1 Gew.-% Indigo Naturalis, 3 Gew.-% Halloysitum Rubrum, 30,3 Gew.-% Katechu, 3 Gew.-% kalzinierten Alaun, 15,2 Gew.-% Rhizoma Bletillae und 3 Gew.-% Galmei umfasst.

4. Zubereitung der Traditionellen Chinesischen Medizin zur Verwendung nach einem der Ansprüche 1 bis 3, wobei die Zubereitung pharmazeutisch zulässigen Träger einschließt und zu einer beliebigen der folgenden Dosierungsformen zubereitet ist: Klistier, Tablette, Kapsel, orale Flüssigkeit, bukkale Tablette, Körnchen, Instantpulver, Pille, Tropfpille, Staub, Paste, Dan, Suspension, Pulver, Lösung, Zäpfchen, Creme, Spray, Trockenpulver-Inhalation, Aerosol, Tropfen, Lutschtablette und Pflaster.

5. Zubereitung der Traditionellen Chinesischen Medizin zur Verwendung beim Vorbeugen und/oder Behandeln extraintestinaler Manifestationen und Komplikationen von Morbus Crohn, wobei die Zubereitung als Rohmaterialien 1-10 Gew.-% Sanguis Draxonis, 15-40 Gew.-% Radix Paeoniae Rubra, 1-20 Gew.-% Indigo Naturalis, 1-10 Gew.- % Halloysitum Rubrum, 15-40 Gew.-% Katechu, 1-10 Gew.-% kalzinierten Alaun, 5-30 Gew.-% Rhizoma Bletillae und 1-10 Gew.-% Galmei umfasst.

6. Zubereitung der Traditionellen Chinesischen Medizin zur Verwendung nach Anspruch 5, wobei die Zubereitung als Rohmaterialien 3-7 Gew.-% Sanguis Draxonis, 25-35 Gew.-% Radix Paeoniae Rubra, 5-15 Gew.-% Indigo Naturalis, 1-5 Gew.-% Halloysitum Rubrum, 25-35 Gew.-% Katechu, 1-5 Gew.-% kalzinierten Alaun, 10-20 Gew.-% Rhizoma Bletillae und 1-5 Gew.-% Galmei umfasst.

7. Zubereitung der Traditionellen Chinesischen Medizin zur Verwendung nach Anspruch 5, wobei die Zubereitung als Rohmaterialien 5,1 Gew.-% Sanguis Draxonis, 30,3 Gew.-% Radix Paeoniae Rubra, 10,1 Gew.-% Indigo Naturalis, 3 Gew.-% Halloysitum Rubrum, 30,3 Gew.-% Katechu, 3 Gew.-% kalzinierten Alaun, 15,2 Gew.-% Rhizoma Bletillae und 3 Gew.-% Galmei umfasst.

8. Zubereitung der Traditionellen Chinesischen Medizin zur Verwendung nach einem der Ansprüche 5 bis 7, wobei die Komplikationen eine oder mehrere Arten von Darmverschluss, Darmfistel, massive untere Magen-Darm-Blutung, Darmperforation, Septikämie, Bauchabszess und Analerkrankungen einschließen.

9. Zubereitung der Traditionellen Chinesischen Medizin zur Verwendung nach einem der Ansprüche 5 bis 7, wobei die extraintestinalen Manifestationen Mundschleimhautentzündung, z.B. Aphthen, pathologische Hautveränderungen, z.B. Knotenrose, Pyoderma gangraenosum (PG) und unspezifisches Exanthem, pathologische Augenveränderungen, z.B. Iridozyklitis und Konjunktivitis, Gelenkschaden, z.B. Gelenkschmerzen und Arthritis, Leber-Galle- Schaden, z.B. Leberfunktionsstörung und Gallenstein, einschließen.

10. Zubereitung der Traditionellen Chinesischen Medizin zur Verwendung nach einem der Ansprüche 5 bis 7, wobei die Zubereitung pharmazeutisch zulässigen Träger einschließt und zu einer beliebigen der folgenden Dosierungsformen zubereitet ist: Klistier, Tablette, Kapsel, orale Flüssigkeit, bukkale Tablette, Körnchen, Instantpulver, Pille, Tropfpille, Staub, Paste, Dan, Suspension, Pulver, Lösung, Zäpfchen, Creme, Spray, Trockenpulver-Inhalation, Aerosol, Tropfen, Lutschtablette und Pflaster.

**Revendications**

1. Préparation de médecine chinoise traditionnelle pour son utilisation dans la prévention et/ou le traitement de la maladie de Crohn, dans laquelle ladite préparation comprend les matières premières suivantes : 1 à 10 % en poids de sang de dragon, 15 à 40 % en poids de racine de pivoine rouge, 1 à 20 % en poids d'indigo naturel, 1 à 10 % en poids d'halloysite rouge, 15 à 40 % en poids de cachou, 1 à 10 % en poids d'alun calciné, 5 à 30 % en poids de rhizome de Bletilla et 1 à 10 % en poids de calamine.

**2.** Préparation de médecine chinoise traditionnelle pour son utilisation selon la revendication 1, dans laquelle ladite préparation comprend les matières premières suivantes : 3 à 7 % en poids de sang de dragon, 25 à 35 % en poids de racine de pivoine rouge, 5 à 15 % en poids d' indigo naturel, 1 à 5 % en poids d'halloysite rouge, 25 à 35 % en poids de cachou, 1 à 5 % en poids d'alun calciné, 10 à 20 % en poids de rhizome de Bletilla et 1 à 5 % en poids de calamine.

**3.** Préparation de médecine chinoise traditionnelle pour son utilisation selon la revendication 1, dans laquelle ladite préparation comprend les matières premières suivantes : 5,1 % en poids de sang de dragon, 30,3 % en poids de racine de pivoine rouge, 10,1 % en poids d'indigo naturel, 3 % en poids d'halloysite rouge, 30,3 % en poids de cachou, 3 % en poids d'alun calciné, 15,2 % en poids de rhizome de Bletilla et 3 % en poids de calamine.

**4.** Préparation de médecine chinoise traditionnelle pour son utilisation selon l'une quelconque des revendications 1 à 3, dans laquelle ladite préparation comprend un vecteur pharmaceutiquement acceptable et est préparée sous l'une quelconque des formes galéniques suivantes : un lavement ; un comprimé, une gélule, un liquide pour administration orale, un comprimé oral, un granule, une poudre instantanée, une pilule, une pilule obtenue par dégouttement, une poudre finement pulvérisée, une pâte, une préparation d'herbes, une suspension, une poudre, une solution, un suppositoire, une crème, une pulvérisation, une poudre sèche pour inhalation, un aérosol, des gouttes, un trochisque et un timbre.

**5.** Préparation de médecine chinoise traditionnelle pour son utilisation dans la prévention et/ou le traitement de manifestations et complications extra-intestinales de la maladie de Crohn, dans laquelle ladite préparation comprend les matières premières suivantes : 1 à 10 % en poids de sang de dragon, 15 à 40 % en poids de racine de pivoine rouge, 1 à 20 % en poids d'indigo naturel, 1 à 10 % en poids d'halloysite rouge, 15 à 40 % en poids de cachou, 1 à 10 % en poids d'alun calciné, 5 à 30 % en poids de rhizome de Bletilla et 1 à 10 % en poids de calamine.

**6.** Préparation de médecine chinoise traditionnelle pour son utilisation selon la revendication 5, dans laquelle ladite préparation comprend les matières premières suivantes : 3 à 7 % en poids de sang de dragon, 25 à 35 % en poids de racine de pivoine rouge, 5 à 15 % en poids d'indigo naturel, 1 à 5 % en poids d'halloysite rouge, 25 à 35 % en poids de cachou, 1 à 5 % en poids d'alun calciné, 10 à 20 % en poids de rhizome de Bletilla et 1 à 5 % en poids de calamine.

**7.** Préparation de médecine chinoise traditionnelle pour son utilisation selon la revendication 5, dans laquelle ladite préparation comprend les matières premières suivantes 5,1 % en poids de sang de dragon, 30,3 % en poids de racine de pivoine rouge, 10,1 % en poids d'indigo naturel, 3 % en poids d'halloysite rouge, 30,3 % en poids de cachou, 3 % en poids d'alun calciné, 15,2 % en poids de rhizome de Bletilla et 3 % en poids de calamine.

**8.** Préparation de médecine chinoise traditionnelle pour son utilisation selon l'une quelconque des revendications 5 à 7, dans laquelle lesdites complications comprennent une ou plusieurs sortes d'une obstruction intestinale, d'une fistule intestinale, d'une hémorragie massive gastro- intestinale basse, d'une perforation intestinale, d'une septicémie, d'un abcès abdominal et de maladies anales.

**9.** Préparation de médecine chinoise traditionnelle pour son utilisation selon l'une quelconque des revendications 5 à 7, dans laquelle lesdites manifestations extra-intestinales comprennent une mucosité bucale, par exemple, la stomatite aphteuse ; des modifications pathologiques de la peau, par exemple, l'érythème noueux, l'idiophagédénisme (PG) et d'une éruption cutanée non spécifique ; des modifications pathologiques des yeux, par exemple, l'iridocyclite et la conjectivite, une lésion articulaire, par exemple, la douleur articulaire et l'arthrite ; une lésion hépatobiliaire, par exemple, une fonction hépatique anormale et un calcul biliaire.

**10.** Préparation de médecine chinoise traditionnelle pour son utilisation selon l'une quelconque des revendications 5 à 7, dans laquelle ladite préparation comprend un vecteur pharmaceutiquement acceptable et est préparée sous l'une quelconque des formes galéniques suivantes : un lavement ; un comprimé, une gélule, un liquide pour administration orale, un comprimé oral, un granule, une poudre instantanée, une pilule, une pilule obtenue par dégouttement, une poudre finement pulvérisée, une pâte, une préparation d'herbes, une suspension, une poudre, une solution, un suppositoire, une crème, une pulvérisation, une poudre sèche pour inhalation, un aérosol, des gouttes, un trochisque et un timbre.

Figure 1

Figure 2

Figure 3

Figure 4

Figure 5

Figure 6

Figure 7

Figure 8

Figure 9

Figure 10

Figure 11

Figure 12

Blank control group

Model group

Positive control group

Low-dose group

Middle-dose group

High-dose group

Figure 13

Blank control group

Model group

Positive control group

Low-dose group

Middle-dose group

High-dose group

Figure 14

Figure 15

Figure 16

Figure 17

Figure 18

Blank control group

Model group

Positive control group

Low-dose group

Middle-dose group

High-dose group

Figure 19

Blank control group

Model group

Positive control group

Low-dose group

Middle-dose group

High-dose group

Figure 20

**EP 3 015 111 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102716383 A
- CN 102048728 A

- CN 1192787 C